# EUROPEAN PATENT APPLICATION

(11) **EP 3 287 135 A1**
(43) Date of publication of application: **28.02.2018**
(21) Application number: 16768689.8
(22) Date of filing: 18.03.2016
(51) Int. Cl.: A61K 35/17, A61K 9/08, A61K 31/7105, A61P 35/00, A61P 35/04, A61P 43/00, C12N 5/078

(54) **THERAPEUTIC AGENT ASSOCIATED WITH SUPPRESSION OF PROLIFERATION AND METASTASIS OF TUMOR, WHICH COMPRISES EXOSOMES RELEASED FROM CYTOTOXIC T CELLS AND TARGETS CANCER STROMAL/MESENCHYMAL CELLS**

(30) Priority: 20.03.2015 JP 2015058467
(71) Applicant: Mie University, Tsu-shi, Mie 514-8507 (JP); Kyoto University, Kyoto-shi, Kyoto 606-8501 (JP)
(72) Inventor: SHIKU, Hiroshi, Tsu-shi Mie 514-8507 (JP); SEO, Naohiro, Tsu-shi Mie 514-8507 (JP); AKIYOSHI, Kazunari, Kyoto-shi Kyoto 606-8501 (JP); HARADA, Naozumi, Tsu-shi Mie 514-8507 (JP); MOMOSE, Fumiyasu, Tsu-shi Mie 514-8507 (JP)
(74) Representative: Sutcliffe, Nicholas Robert
(86) International application number: PCT/JP2016/058721
(87) International publication number: WO 2016/152786

(57) **Abstract**

[Problem to be solved] To provide a therapeutic agent which can suppress cell proliferation and metastasis of a tumor or the like.

[Solution] Provided is a therapeutic agent for cell-proliferative diseases, which comprises extracellular vesicles (exosomes) released from cytotoxic T cells. The therapeutic agent targets cancer stromal / mesenchymal cells and can suppress the proliferation and metastasis of a tumor including cancer.

## Description

### [Technical Field]

The present invention relates to a therapeutic agent for treating cell-proliferative diseases including cancers, the therapeutic agent including exosomes released from cytotoxic T cells or miRNA contained in the exosomes as active ingredients.

### [Background Art]

In primary tumor tissues, extracellular matrices such as fibronectin, laminin and collagen and many types of other cells together constitute tumor-associated stroma. These cells include cancer-associate fibroblasts (CAFs; fibroblast markers and platelet-derived growth factor receptor α (CD140a)⁺, α smooth muscle actin (α-SMA)⁺) and mesenchymal stem cells (MSCs; (see Non Patent Document 1, the references are listed at the end of the specification): CD140a⁺, stem cell antigen (Sca-1)⁺). In addition, cancer cells are rigidly adhered each other through E-cadherin or the like (Non Patent Documents 3 and 4), and after gaps between the cancer cells are filled by growing stromal cells, angiogenesis occurs there (Sca-1⁺CD31⁺) (Non Patent Document 2). Malignant transformation of the cancer cells, exemplified by the epithelial to mesenchymal transition (EMT) by interaction with the stromal cells, plays an important role for invasion and metastasis of tumors. The epithelial to mesenchymal transition can be a marker to determine whether tumors are under malignant transformation (Non Patent Document 5). Molecules responsible for the epithelial to mesenchymal transition have been reported in some documents (Non Patent Documents 3, 6, and 7).

Endosomal membrane-derived microvesicles (having diameters of 100 nm to 200 nm) are released from many types of cells including tumor cells and tumor-associated stromal cells, and proteins and RNA contained therein work for cell-cell signaling (Non Patent Documents 8 and 9). Tumor cells release a variety of extracellular vesicles (ECV) (sometimes referred to as exosomes, and will be herein described as "exosomes" or "ECV") that have been reported to be responsible for self-proliferation, immune tolerance, adjustments of tumor environments and the like (Non Patent Documents 8, and 10 to 13). Meanwhile, some Documents have reported that exosomes released from tumors promote epithelial to mesenchymal transitions, expansions, and aggravations of tumors (Non Patent Documents 9, 14, and 15). Accordingly, researches of exosomes have become important to evaluate aggravations of tumors.

According to researches regarding mouse models and humans, activated tumor-invading CD8⁺ T cells seem to invade in tumors and tumor-associated stroma (Non Patent Document 16). Furthermore, in immunotherapies using monoclonal antibodies against tumor-associated antigens, CD8⁺ T cells including cytotoxic T-lymphocytes (CTL) accumulate to, or proliferate in tumor tissues (Non Patent Documents 17 and 18). Although CTLs are not tumor specific, they invade in tumors through blood vessels by remodeling basement membranes (Non Patent Document 19). Thus, CD8⁺ T cells are supposed to be responsible for growth and aggravations of tumors in various ways.

### [SUMMARY OF THE INVENTION]

### [Problems to be Solved by the Invention]

To date, however, little has been known about exosomes released from cytotoxic T cells for what roles they play on malignant transformations of tumors under what conditions.

Then, the inventors of the present invention have investigated effects of the exosomes released from cytotoxic T cells to malignant transformations of tumors in detail. As a result, the inventors of the present invention have found that exosomes derived from CD8⁺ T cells do not kill a cancer portion of tumor tissues, but kill mesenchymal cells around the tumor tissues to suppress progression of cancers including proliferation and metastasis. This can be observed particularly in exosomes derived from CD8⁺ T cells among other cytotoxic T cells. Based on these findings, the present invention has been basically completed.

A first aspect of the present invention relates to a therapeutic agent for cell-proliferative diseases, the therapeutic agent including extracellular vesicles (exosomes) released from cytotoxic T cells.

A second aspect of the present invention relates to the therapeutic agent for cell-proliferative diseases according to the first aspect, in which the cytotoxic T cells include extracellular vesicles (exosomes) released from at least one of human CD4⁺, CD8⁺, CD9⁺, CD63⁺, and TCR⁺ T cells.

A third aspect of the present invention relates to the therapeutic agent for cell-proliferative diseases according to the second aspect, in which the cytotoxic T cells include the extracellular vesicles (exosomes) released from CD8⁺ T cells.

A fourth aspect of the present invention relates to the therapeutic agent for cell-proliferative diseases according to the second or the third aspect, in which the extracellular vesicles (exosomes) include miRNA effective in suppressing cell proliferation.

A fifth aspect of the present invention relates to the therapeutic agent for cell-proliferative diseases according to the fourth aspect, the therapeutic agent including the miRNA effective in suppressing cell proliferation.

A sixth aspect of the present invention relates to the therapeutic agent for cell-proliferative diseases according to the second aspect, the therapeutic agent further including one or more selected from microbicides, mucosa removing agents, tonicity agents, pH control agents, stabilizing agents, thickening agents, preservatives, adhesives, and immune enhancement agents.

A seventh aspect of the present invention relates to the therapeutic agent for cell-proliferative diseases according to the second aspect, the therapeutic agent being injected to tumor tissues, to mesenchymal cells residing inside tumor tissues, intravenously or subcutaneously.

An eighth aspect of the present invention relates to a method of extracting miRNA for treating cell-proliferative diseases, the method including collecting exosomes released from cytotoxic T cells; and identifying miRNA effective in suppressing cell proliferation in the exosomes.

A ninth aspect of the present invention relates to a therapeutic agent for cell-proliferative diseases, the therapeutic agent including miRNA effective in suppressing cell proliferation.

A tenth aspect of the present invention relates to a method of identifying MSC cytotoxic miRNA, the method including adding miRNA having a base sequence that is same as a base sequence of miRNA contained in exosomes released from cytotoxic T cells to cultured human mesenchymal stem cells (MSCs), culturing the MSC, and investigating toxic activity to the MSC to evaluate MSC cytotoxicity of the miRNA.

Once MSC cytotoxic miRNA is identified, exosomes containing the miRNA or miRNA having a sequence same as the sequence of the identified miRNA are synthesized, and the resulting miRNA is reconstituted as it is or like exosomes to use as a therapeutic agent for cell-proliferative disorders. Thus, an eleventh aspect of the present invention relates to a therapeutic agent for cell-proliferative diseases, the therapeutic agent including MSC cytotoxic miRNA.

Another aspect relates to a method for treating cell-proliferative diseases, the method including administering the therapeutic agent of each of the aspects described above to a patient. An administration method is preferably any of an intratumoral method to tumor tissues, an intracellular method to mesenchymal cells residing inside tumor tissues, an intravenous method or a subcutaneous method.

The exosome refers to a vesicle composed of a lipid bilayer membrane externally secreted from a variety of cells, which has a diameter of about 40 nm to 200 nm. In biological bodies, exosomes can be observed in body fluids such as saliva, blood, urine, amniotic fluids, and malignant ascites. In addition, exosomes are secreted from culture cells to culture media. A variety of proteins and RNA are contained in exosomes, and they have been thought to work for cell-cell signaling.

Moreover, according to the present invention, by administering exosomes obtained from cytotoxic T cells, the exosomes kill mesenchymal cells around cancer cells (cancer stromal disintegration) so that proliferation/metastasis of the cancer cells can be suppressed. Among those cytotoxic T cells, exosomes derived from CD8⁺ T cells are particularly effective in suppressing proliferation/metastasis. The mechanism of the action is as follows. When the exosomes are incorporated in both of the cancer cells and mesenchymal cells, only the mesenchymal cells will die (apoptosis). As a result, proliferation/metastasis of cancer cells will be suppressed because stromal cells that are necessary for proliferation and metastasis of cancer cells have been lost and the cancer cells are isolated. These suppressive effects of proliferation/metastasis of cancer cells can also be observed by using specific miRNA contained in the exosomes.

### [Effects of the Invention]

The present invention can provide a therapeutic agent for cell-proliferative diseases, the therapeutic agent including extracellular vesicles (exosomes) derived from cytotoxic T cells as active ingredients. Furthermore provided is a therapeutic agent for cell-proliferative diseases, the therapeutic agent including miRNA effective for suppression of cell proliferation. These therapeutic agents according to the present invention can suppress cell proliferation and metastasis of tumors such as cancer.

### [BRIEF DESCRIPTION OF THE FIGURES]

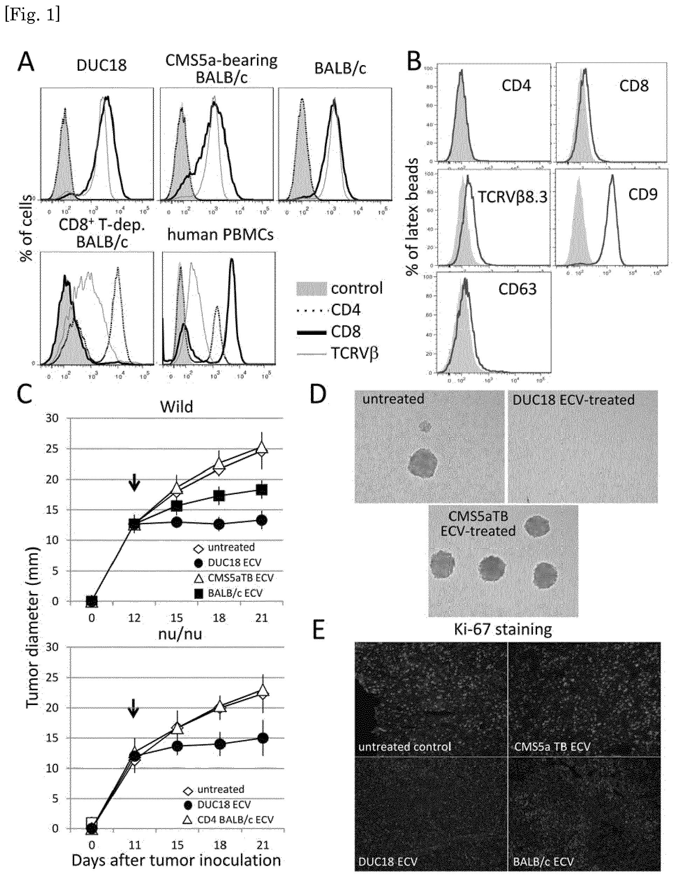

FIG. 1 illustrates suppressive effects of tumor proliferation, which was induced by intratumoral administrations of ECV released from CD8⁺ T cells. A. Graphs illustrate results of flow cytometry analyses of 4 day-cultured splenocytes obtained from DUC18, CMS5a-inoculated BALB/c, BALB/c, and CD8⁺ T cell deficient BALB/c, as well as hPBMC, using monoclonal antibodies specific for CD4⁻, CD8⁻, and TCRαβ⁻ for mice and humans. B. Graphs illustrate results of flow cytometry analyses of DUC18 ECV-immobilized latex beads stained with a control monoclonal antibody and a CD4⁻, CD8⁻, TCRVb⁻, CD9⁻, or CD63⁻ specific monoclonal antibody. C. Graphs illustrate tumor growth. CMS5a was inoculated to wild type mice and BALB/c nude mice. On day 12 after the inoculation of CMS5a, ECV derived from DUC18, CMS5a TB or BLAB/c was intratumorally injected to those mice (The symbols in the graphs indicate the following: *<0.05, **<0.001, n.s.: no significant difference). D. Micrographs of spheroid formations. On day 3 after ECV treatments, suspensions of CMS5a tumors were prepared. The micrographs were taken after culturing the suspensions for 24 hours. E. Fluorescence micrographs of CMS5a tumor sections on day 3 after ECV treatments. The sections were stained with a Ki-67 monoclonal antibody and DAPI.

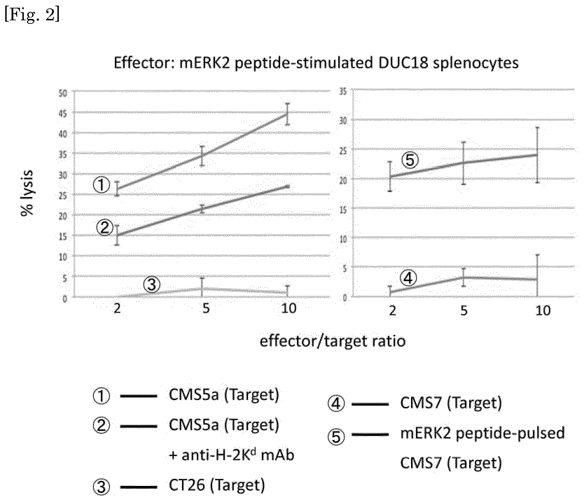

FIG. 2 includes graphs illustrating lytic activities of DUC18 CD8⁺ T cells specific to the corresponding tumors. Cytotoxicity assays were conducted for DUC18 CD8⁺ T cells stimulated with mutated ERK2 peptide, by using CMS5a, H-2Kd-neutralizing CMS5a, CT26, CMS7, and mERK2 peptide-pulsed CMS7 as target cells.

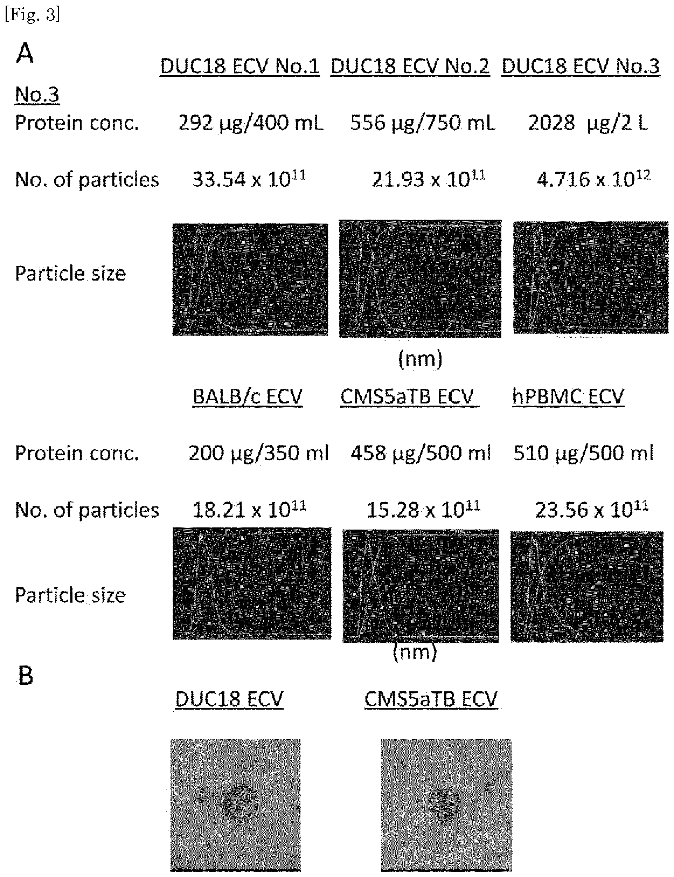

FIG. 3 illustrates the total protein concentrations and the total numbers and average diameters of particles of ECV used in this study. A. Three lots of ECV derived from DUC18, BALB/c, CMS5a TB, or hPBMC were measured for total protein concentrations (values) by the BCA assay, and for the total numbers (values) and average diameters (graphs) by the NTA assay. B. Electron micrographs of DUC18 ECV and CMS5a ECV.

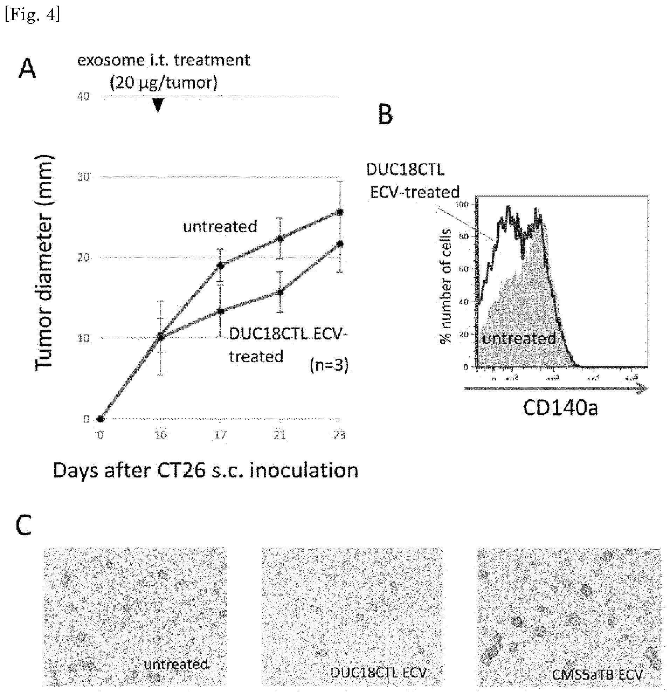

FIG. 4 illustrates downregulation of CT26 tumor proliferation by DUC18 ECV administration. A. On day 10 after the administration, DUC18 ECV was intratumorally injected to CT26-inoculated BALB/c mice, and tumor diameters were recorded as illustrated in this graph. B. CT26 tumor cells were co-cultured with cultured BM-MSC under the presence or absence of DUC18 ECV, and expressions of CD140a were analyzed by flow cytometry as illustrated in this graph. C. CT26 tumor cells were co-cultured with cultured BM-MSC under the presence of DUC18 ECV or CMS5a TB ECV, and micrographs were taken to check spheroid formations.

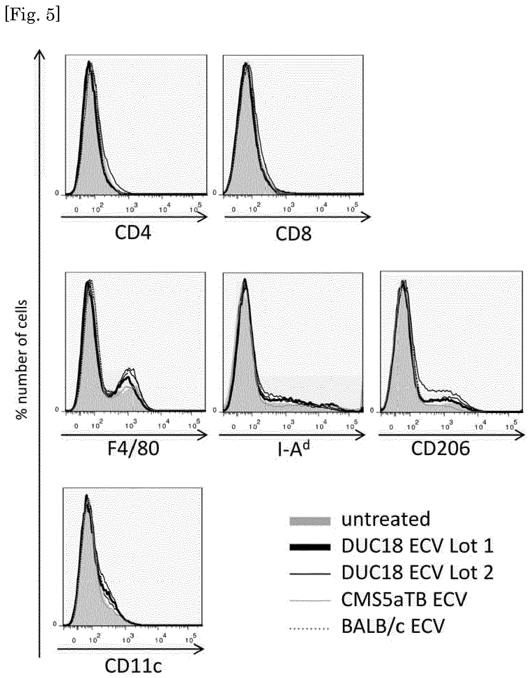

FIG. 5 includes graphs illustrating ECV derived from CD8⁺ T cells not regulating abundance ratios of tumor T lymphocytes, macrophages, and dendritic cells. Two lots of DUC18 ECV, CMS5a TB ECV or BALB/c ECV were intratumorally injected to CMS5a-inoculated BALB/c mice. On day 3 after administration, tumor suspensions were stained with a monoclonal antibody against CD4, CD8, F4/80, I-Ad, CD206, or CD11c, and analyzed by flow cytometry.

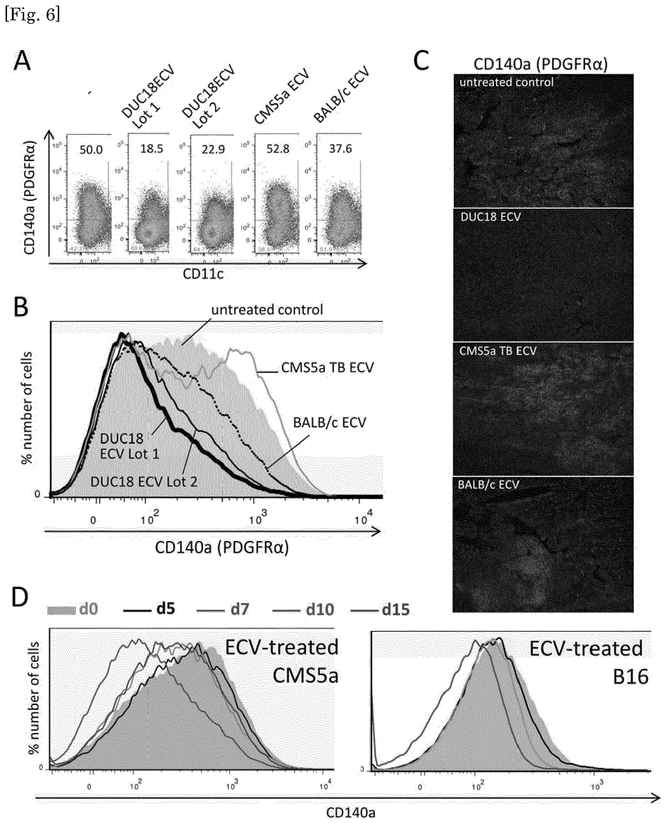

FIG. 6 illustrates downregulation of CD140a (PDGFRα) expressions induced by intratumoral injections of ECV released from CD8⁺ T cells. A and B. Two lots of DUC18 ECV were intratumorally injected to CMS5a tumor. CD140a expressions were analyzed by flow cytometry, and results were compared with a CMS5a ECV treating group and a BALB/c ECV treating group as illustrated in these graphs. (A) is a dot plot, and (B) is a histogram. C. Fluorescence micrographs of CMS5a tumor sections on day 3 after ECV treatments. The sections were stained with a CD140a specific monoclonal antibody and DAPI. D. ECV prepared from culture media of B6 splenocytes stimulated with TRP-2 and gp100 peptides (day 5, day 7, day 10, or day 15) was intratumorally injected to CMS5a-inoculated BALB/c mice or B16-inoculated B6 mice on day 12 after tumor inoculations. On day 3 after ECV injection, suspensions of the tumor cells were stained with a CD140a specific monoclonal antibody, and CD140a expressions of the tumor cells were analyzed by flow cytometry. The results are illustrated in these graphs.

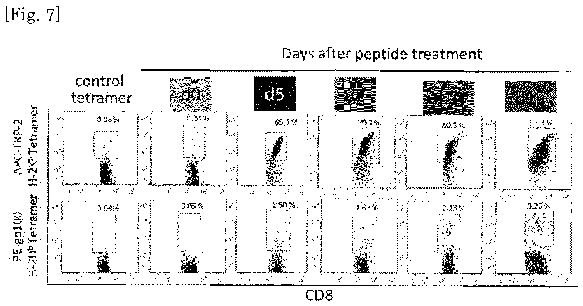

FIG. 7 illustrates results of kinetics of B6 splenocytes stimulated with TRP-2 and gp100 peptides. B6 splenocytes were stimulated with TRP-2 and gp100 peptides. On day 0, day 5, day 7, day 10, and day 15 of stimulation, inductions of TRP-2 or gp100 specific CD8⁺ T cells were analyzed by flow cytometry with corresponding tetramers. The results are illustrated in these graphs. An irrelevant tetramer was used as a control.

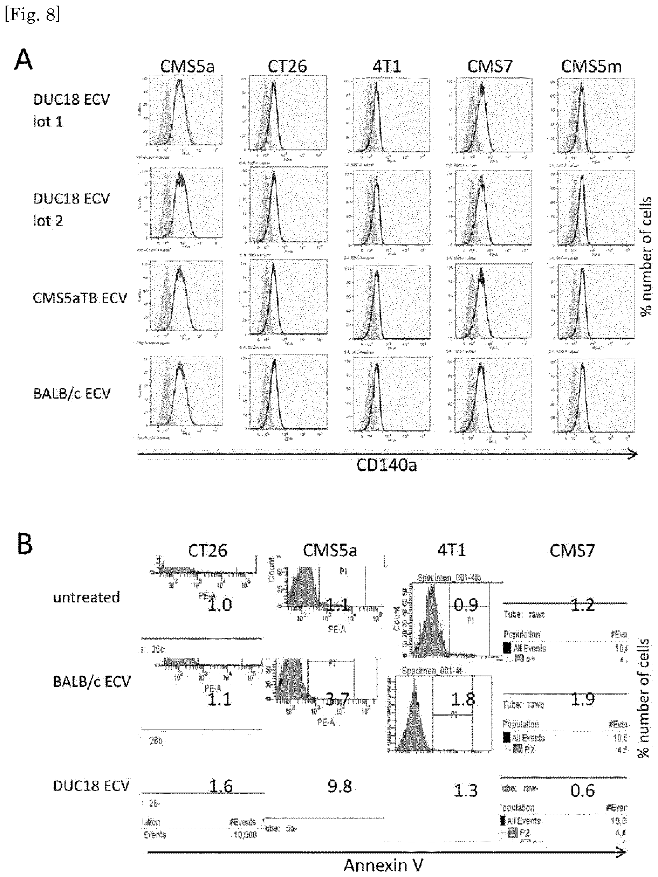

FIG. 8 illustrates ECV derived from CD8⁺ T cells not directly suppressing CD140a expression or apoptosis of cultured tumor cells. A. Two lots of DUC18 MEV, BALB/c ECV, and CMS5a TB ECV were added to culture media of CMD5a, CT26, 4T1, CMS7, or CMS5m. On day 3 after addition, CD140a expression was analyzed for each of the tumors by flow cytometry. The results are illustrated in these graphs. A rat IgG2a monoclonal antibody was used as a control. B. DUC18 ECV and BLAB/c ECV were added to culture media of CT26, CMS5a, 4T1, and CMS7. On day 3 after addition, each of the tumor cells was stained with an annexin V monoclonal antibody, and analyzed by flow cytometry. The results are illustrated in these graphs.

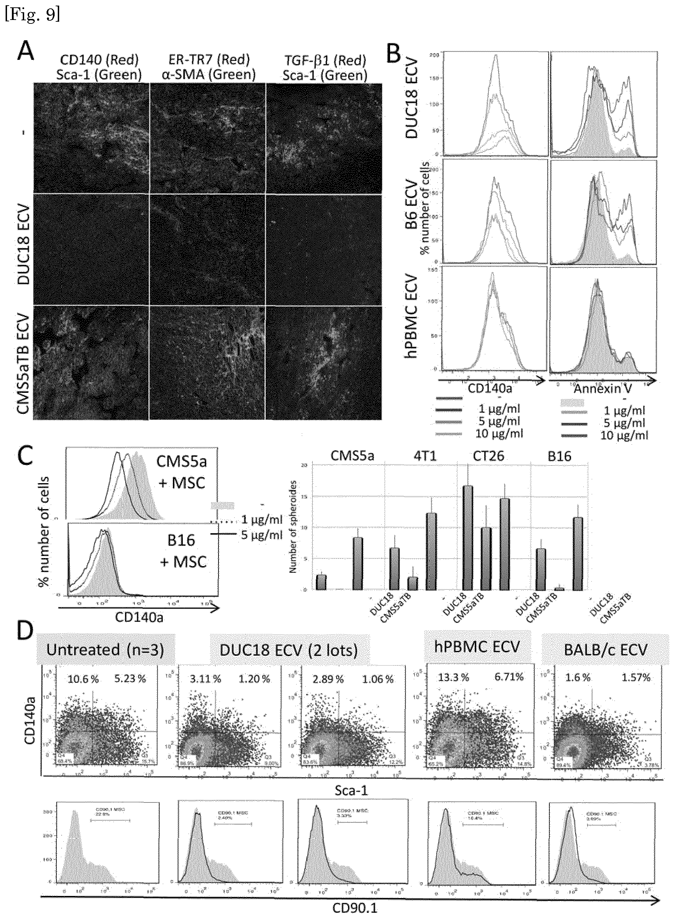

FIG. 9 illustrates ECV released from CD8⁺ T cells inducing apoptosis to BM-MSC, whereby BM-MSC mediated tumor proliferation is suppressed. A. Fluorescence micrographs of sections obtained from CMS5a tumors on day 3 after intratumoral injections of DUC18 ECV or CMS5a TB ECV. The sections were stained with a CD140a specific monoclonal antibody, an Sca-1 specific monoclonal antibody, and DAPI to evaluate tumor proliferation and stromal stem cells (MSCs); stained with an ER-TR7 specific monoclonal antibody, an α-SMA specific monoclonal antibody, and DAPI to evaluate cancer-associate fibroblasts (CAFs); and stained with a TGF-β1 specific monoclonal antibody, an Sca-1 specific monoclonal antibody, and DAPI to evaluate epithelial to mesenchymal transition(EMT) of the tumor. B. Cells obtained from crushed femora were cultured for 1 month to prepare BM-MSC. DUC18, B6, or hPBMC ECV was added to a BM-MSC culture medium with the concentration illustrated in the figure. After 3 days, remaining BM-MSC was stained with an annexin V monoclonal antibody, and the total number was counted by flow cytometry as illustrated in these graphs. C. DUC18 ECV was added to CMS5a or B16 co-cultured with MSC with the concentration illustrated in the figure, and cultured for 4 days. The obtained tumor cells were stained with a CD140a specific monoclonal antibody, and analyzed by flow cytometry. The number of spheroids was determined under a microscope for each of CMS5a, 4T1, CT26, and B16, to which DUC18 ECV or CMS5a TB ECV was added and cultured with MSC (Each of the symbols in the graphs indicate the following: *<0.05, **<0.001, n.s.: no significant difference). The results are illustrated in these graphs. D. CD90.1⁺ BM-MSC chimera mice were generated by transferring normal BM cells of BLAB/c mice and cultured CD90.1⁺ BM-MSC to irradiated BALB/c. In month 2 after CD90.1⁺ BM-MSC was transferred, DUC18 ECV, hPBMC ECV, or BLAB/c ECV was injected into CMS5a tumor that was on day 12 after inoculation. On day 3 after ECV treatment, percentages of CD140a and Sca-1 positive cells and CD90.1 expression were analyzed by flow cytometry as illustrated in these graphs.

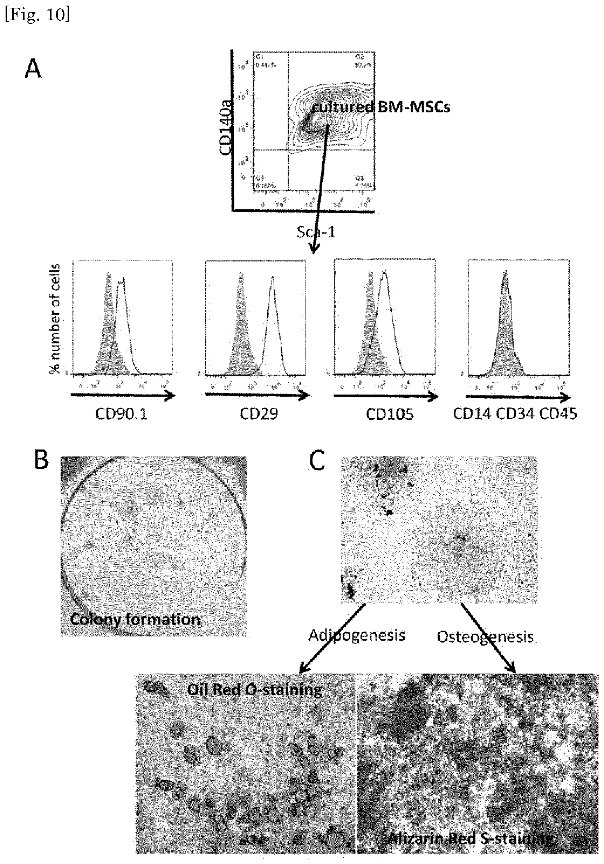

FIG. 10 illustrates characterization of cultured CD90.1 BM-MSC. A. The CD90.1 BM-MSC cultured for 2 months were stained with a mixture of a PE-conjugated CD140a monoclonal antibody, an APC-conjugated Sca-1 monoclonal antibody, an FITC-conjugated CD90.1, CD29, or CD105 monoclonal antibody, or CD14, CD34, and CD45 monoclonal antibodies. The obtained cells were analyzed by flow cytometry as illustrated in these graphs. B. A photograph of Giemsa staining to determine colony formation of BM-MSC after culturing for 2 weeks. C. The BM-MSC cultured for 1 month (about 80% confluent) was further cultured in a fat formation medium or a bone formation medium for 3 weeks to induce differentiation. Photographs of BM-MSC differentiated into adipocytes or osteocytes are illustrated. BM-MSC was stained with Oil Red O or Alizarin Red S.

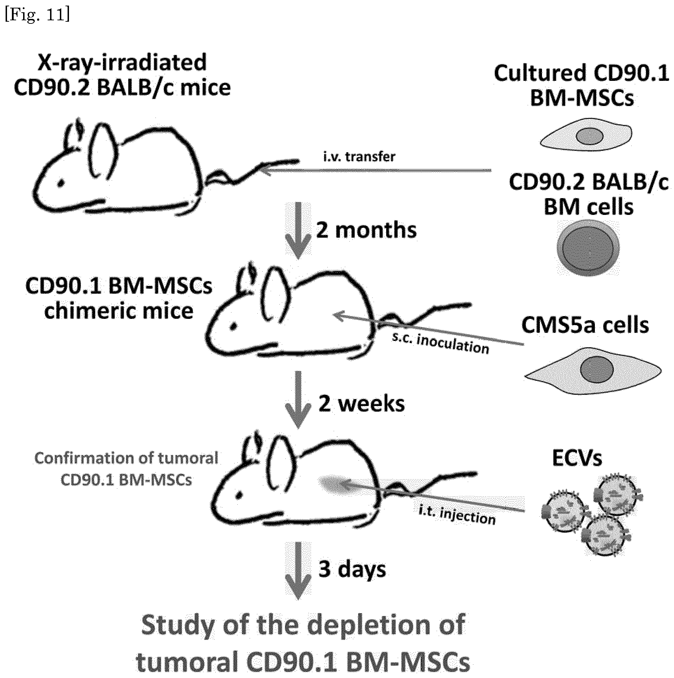

FIG. 11 is a schematic diagram explaining the strategy to generate CD90.1 BM-MSC chimera BALB/c mice. These mice were used to clarify the role of ECV released from CD8⁺ T cells on apoptosis of tumor-invading MSC. Six Gy radiation was given to BALB/c mice, and cultured CD90.1 BM-MSC and BALB/c BM cells were intravenously transferred to these mice. In month 2 after cell transfer, CMS5a tumor cells were subcutaneously inoculated to the CD90.1 BM-MSC chimera mice. In week 2 after CMS5a inoculation, DUC18 ECV, BALB/c ECV, or hPBMC ECV was injected into CMS5a tumor (having a diameter of about 1 cm). On day 3 after ECV injection, obtained splenocytes were collected, and depletion of the tumoral CD90.1⁺ cells was analyzed by flow cytometry.

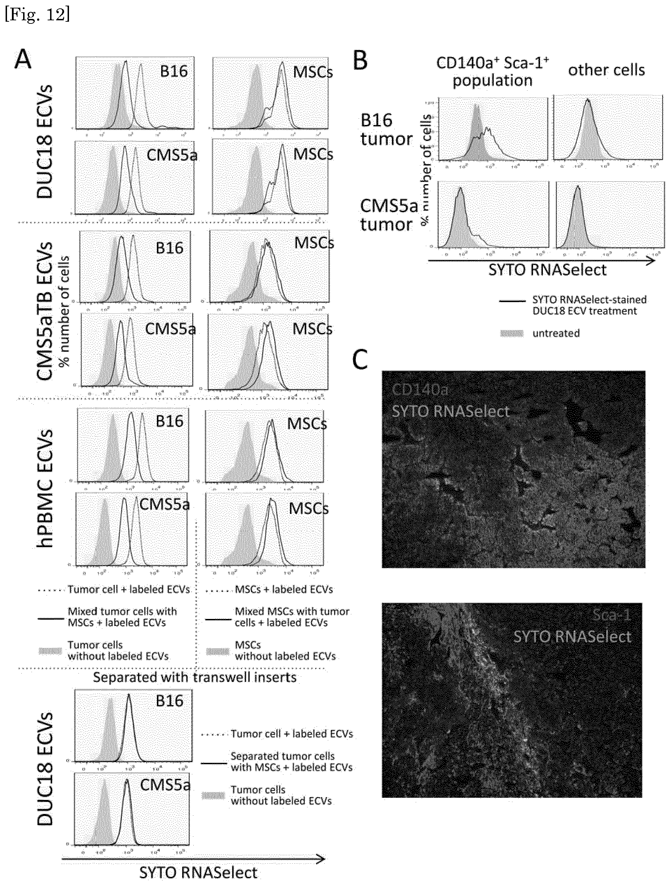

FIG. 12 illustrates ECV-derived RNA being functionally incorporated into BM-MSC, but not into tumor cells. A. B16 cells or CMS5a cells and BM-MSC were co-cultured or individually cultured for 3 days, and then SYTO RNASelect-stained DUC18, CMS5a TB or hPBMC ECV was added thereto. Strengths of green fluorescence emitted from SYTO RNASelect on B16, CMS5a, or BM-MSC were analyzed by flow cytometry 2 hours after ECV was added, as illustrated in these graphs. Untreated tumor cells and BM-MSC were used as negative controls. B16, CMS5a, or BM-MSC treated with SYTO RNASelect-stained ECV was individually used as a positive control. B. B16 or CMS5a tumor suspensions were prepared 30 minutes after SYTO RNASelect-stained DUC18 ECV was added, and the suspensions were stained with CD140a, Sca-1 specific monoclonal antibodies, and analyzed by flow cytometry as illustrated in these graphs. C. Sections were prepared from tumors 30 minutes after SYTO RNASelect-stained DUC18 ECV was injected. Fluorescence micrographs of the sections stained with a CD140a specific monoclonal antibody and DAPI, or Sca-1 specific monoclonal antibody and DAPI.

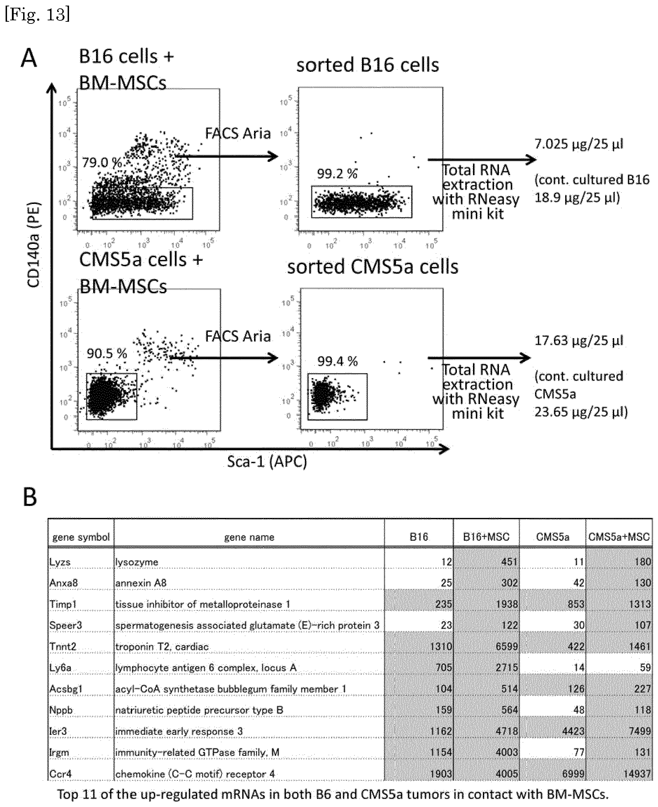

FIG. 13 illustrates results of the total mRNA microarray analyses of tumor cells contacted with cultured BM-MSC. A. B16 cells or CMS5a cells were cultured individually or co-cultured with cultured BM-MSC for 3 days. Tumor cells were separated from BM-MSC by using a fluorescence-activated cell sorter. Total RNA was extracted from tumor cells co-cultured with BM-MSC or tumor cells cultured alone, and subjected to a total mRNA microarray analysis using the RNeasy mini kit as illustrated in this graph. B. The top 9 genes whose expressions were increased in both B16 and CMS5a tumors co-cultured with BM-MSC are illustrated.

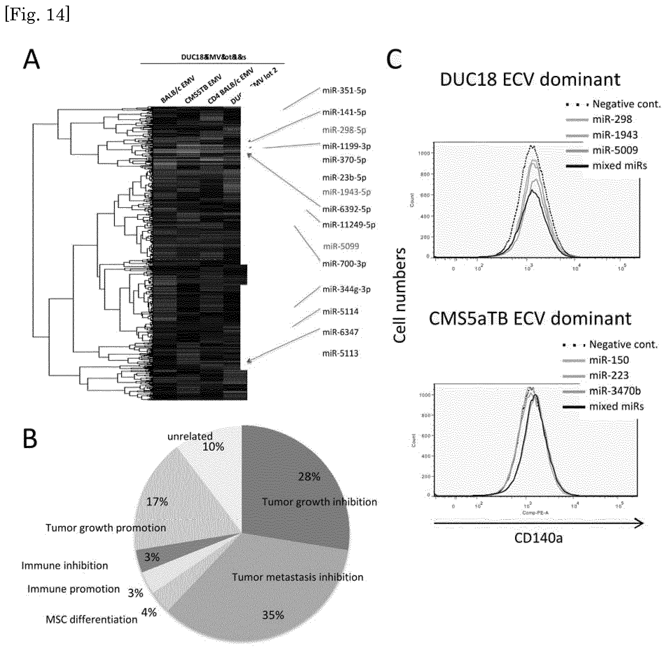

FIG. 14 illustrates miRNA involved in depletion of cultured BM-MSC. A. miRNA was obtained from ECV released from BALB/c, CMS5a TB, and CD4 BALB/c, and compared them to select miRNA dominant in DUC18 ECV. The selected 15 miRNA genetically form 2 clusters. miR-298, miR-1943, and miR-5099 (indicated in red) were higher on their expressions, and obtained from those unknown in PubMed. B. miR-351, -700, -1943, -344g, -1199, -5113, -5114, -6347, - 6392, and -5099 were not known in PubMed search in the fields of tumor, cancer, immune system, invasion or metastasis. miR-298, -141, -1249, -23b, and -370 have been reported to be involved in tumor proliferation and immune activation, as well as tumor proliferation and immunostimulation. As illustrated in the pie graph, 70% of existing reports regarding the selected DUC18 ECV dominant miRNA were about downregulation of tumor promotion and proliferation. The fact proves that the search made by the inventors of the present invention is accurate. C. The selected miR-298, -1943, and -5099 were synthesized, and were transfected to cultured BM-MSC individually or as mixed. Negative control miR and synthesized CMS5a TB ECV dominant miR (miR-150, -223, or -3470b) were used as controls. On day 3 after transfection, the total number of remaining BM-MSC was counted by flow cytometry as illustrated in these graphs.

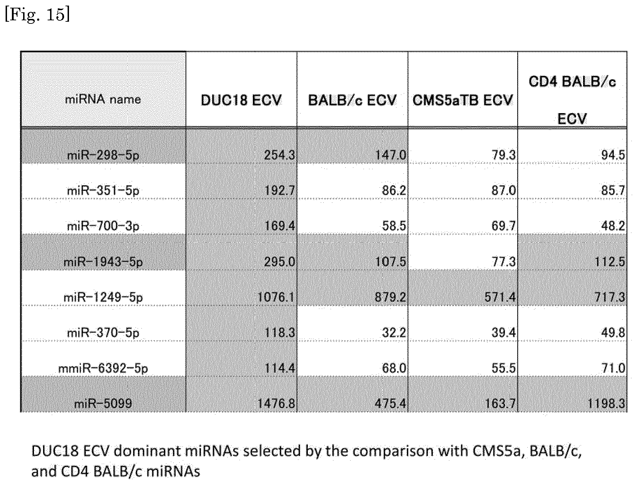

FIG. 15 illustrates 14 DUC18 ECV dominant miRNA selected by comparing with BLAB/c, CMS5a TB, or CD4 BALB/c miRNA. miRNA having 100 indicators or more, extracted by global normalization, are indicated in orange. Three miRNAs indicated in gray (miR-298-5p, -1943-5p, -5099) were used for studying BM-MSC depletion.

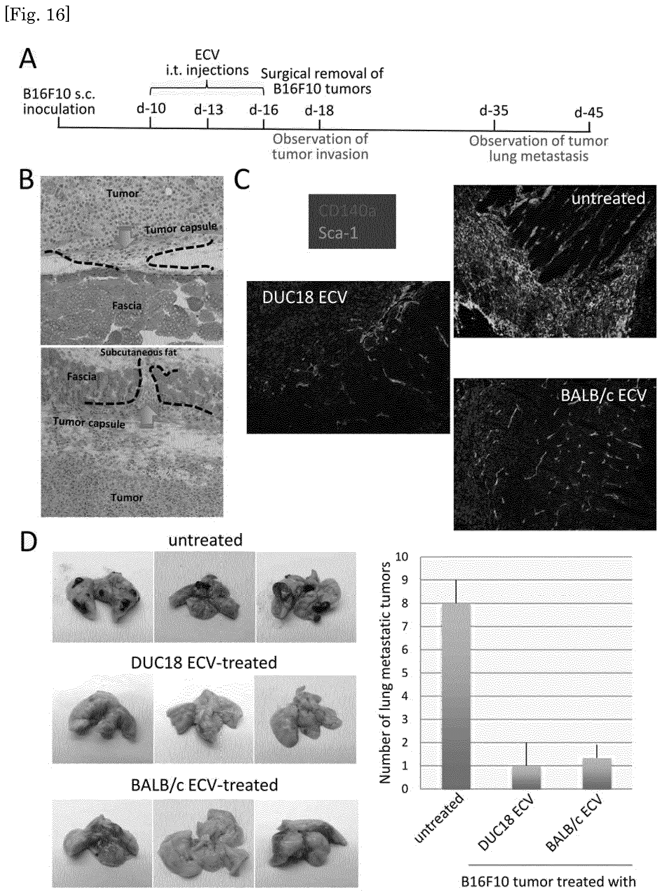

FIG. 16 illustrates invasion and metastasis of B16F10 being suppressed by injecting DUC18 ECV and BALB/c ECV to primary tumors. A. A diagram illustrating the time points of the following: B16F10 melanoma cells were subcutaneously injected to B6 mice, and then invasion and lung metastasis of B16F10 were sequentially observed. On day 10, day 13, and day 16 after tumor injection, DUC18 or BALB/c ECV was injected into primary B16F10 tumor with 50 µg/tumor/site. B. On day 18 after tumor injection, the B16F10 tumor was removed, and invasion was investigated by HE staining. Representative micrographs of 6 tumors in the untreated group are illustrated. C. On day 18 after tumor injection, sections of the B16F10 tumor were prepared, and stained for CD140a, Sca-1 and DAPI. Representative fluorescence micrographs of 3 samples in each group are illustrated. D. On day 45 after tumor injection, lung metastasis of the B16F10 tumor (photographs) and the number of metastasized tumors (graph) were investigated for each group (*<0.05, **<0.001).

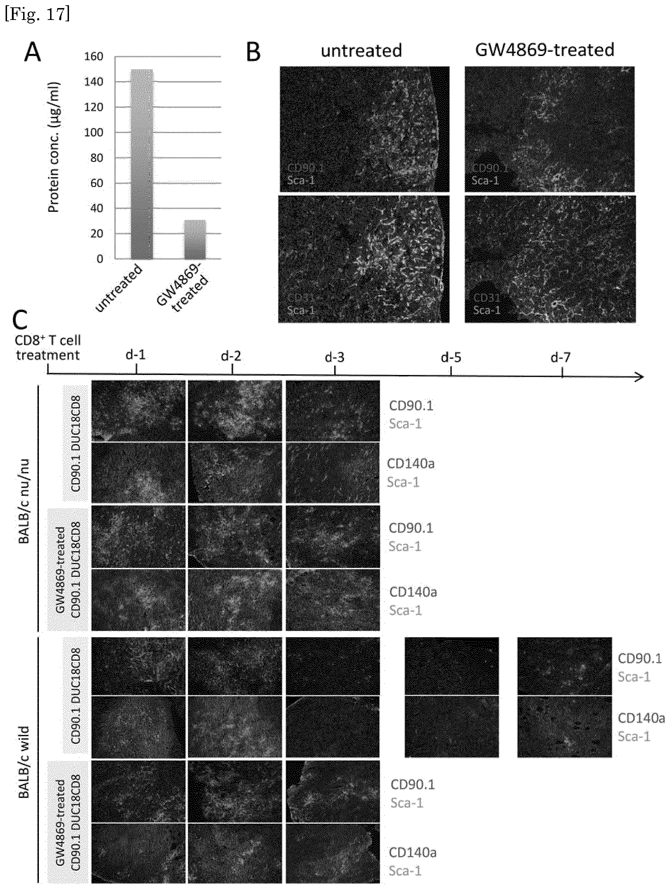

FIG. 17 illustrates CD8⁺ T cells invaded from an angiogenic site of tumor lesion capable of destroying formation of tumor-associated stroma through ECV produced from these cells. A. CD90.1 DUC18 CD8⁺ T cells treated with DMSO (untreated) or GW4869 were cultured. Total protein concentrations of ECV obtained from these cells were measured by the BSA method. The result is illustrated in this graph. B. CD90.1 DUC18 CD8⁺ T cells treated with DMSO or GW4869 were intravenously injected to BALB/c mice on day 12 after CMS5a inoculation. After 24 hours, sections of the obtained tumor were prepared. The fluorescence micrographs illustrate the sections stained with a CD90.1 monoclonal antibody, an Sca-1 monoclonal antibody, and DAPI, or a CD31 monoclonal antibody, an Sca-1 monoclonal antibody, and DAPI. C. The CMS5a tumor was inoculated to BALB/c wild type or nude mice. On day 12 after inoculation, CD90.1 DUC18 CD8⁺ T cells treated with DMSO or GW4869 were injected to these mice. On day 1, day 2, day 3, day 5, and day 7 after injection, sections of the tumor were prepared. The fluorescence micrographs illustrate the sections stained with a CD90.1 monoclonal antibody, an Sca-1 monoclonal antibody, and DAPI, or a CD140a monoclonal antibody, an Sca-1 monoclonal antibody, and DAPI.

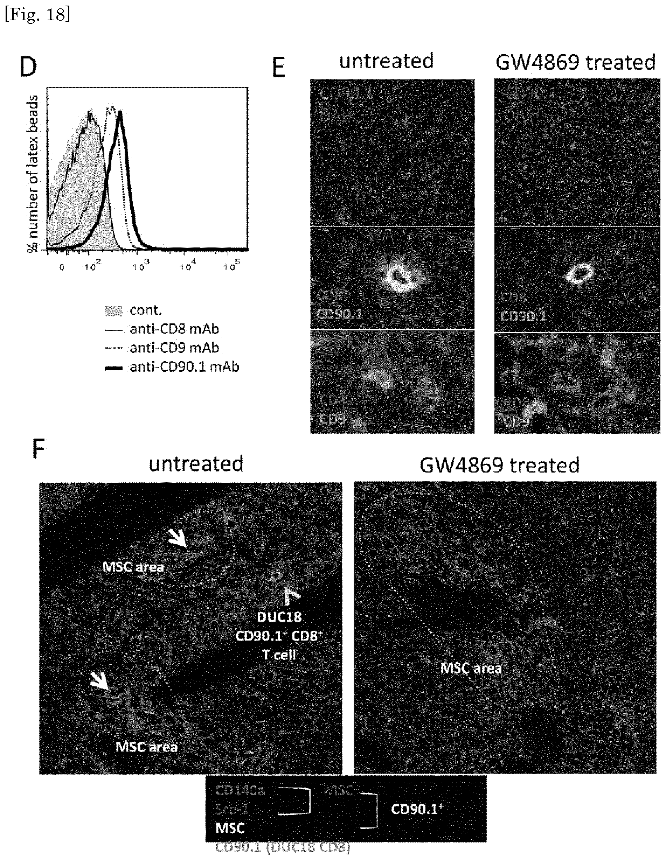

FIG. 18 illustrates CD8⁺ T cells invaded from an angiogenic site of tumor lesion capable of destroying formation of tumor-associated stroma through ECV produced from these cells. D. ECV obtained from culture supernatants of CD90.1 DUC18 CD8⁺ T cells was immobilized on latex beads, and stained with a control monoclonal antibody, a CD8 monoclonal antibody, a CD9 monoclonal antibody, or a CD90.1 monoclonal antibody to analyze by flow cytometry as illustrated in this graph. E. Sections of the tumor were obtained 24 hours after injection of GW4869-treated or untreated CD90.1 DUC18 CD8⁺ T cells. The fluorescence micrographs illustrate the sections stained with a CD90.1 (Thy-1.1) monoclonal antibody and DAPI; a CD8 monoclonal antibody, a CD90.1 monoclonal antibody, and DAPI; or a CD8 monoclonal antibody, a CD9 monoclonal antibody, and DAPI. F. Sections of the tumor were obtained 24 hours after injection of GW4869-treated or untreated CD90.1 DUC18 CD8⁺ T cells, stained with an FITC-conjugated CD90.1 (Thy-1.1) monoclonal antibody, a PE-conjugated CD140a monoclonal antibody, and an APC-conjugated Sca-1 monoclonal antibody, and DAPI, and observed under a 2 photon confocal microscope. The MSC areas stained in purple are circled with dots. In the fluorescence micrographs, yellow arrows indicate CD90.1 DUC18 CD8⁺ T cells invaded into the tumor, and white arrows indicate CD140a⁺ Sca-1⁺ MSC uptaking CD90.1 ECV. Representative focal points from 6 photographs are illustrated.

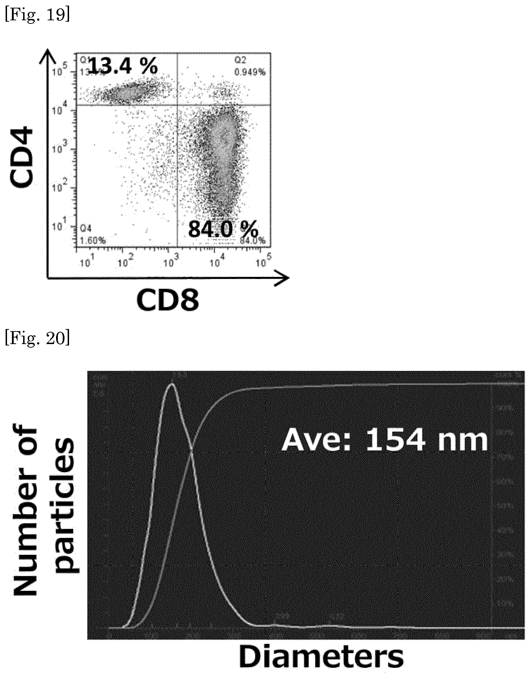

FIG. 19 illustrates the result of flow cytometry indicating CD4⁺ and CD8⁺ of the T cell population obtained from monocytes that were isolated from human peripheral blood, and cultured for 2 weeks.
FIG. 20 is a graph illustrating results of investigation in terms of diameters of exosomes released from human T cells.

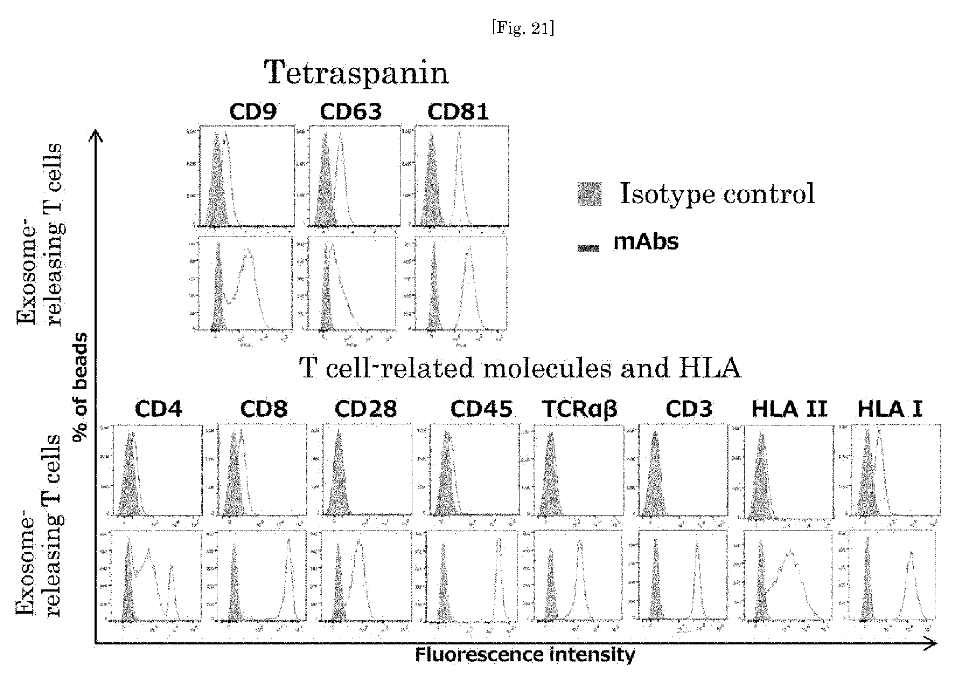

FIG. 21 is a graph illustrating the results of flow cytometry analyses of surface molecules on exosomes released from human T cells and those on human T cells. The horizontal axes of these graphs indicate fluorescence intensities, and vertical axes indicate percentages of the beads (%). The analyzed molecules are illustrated at the top of the graphs.

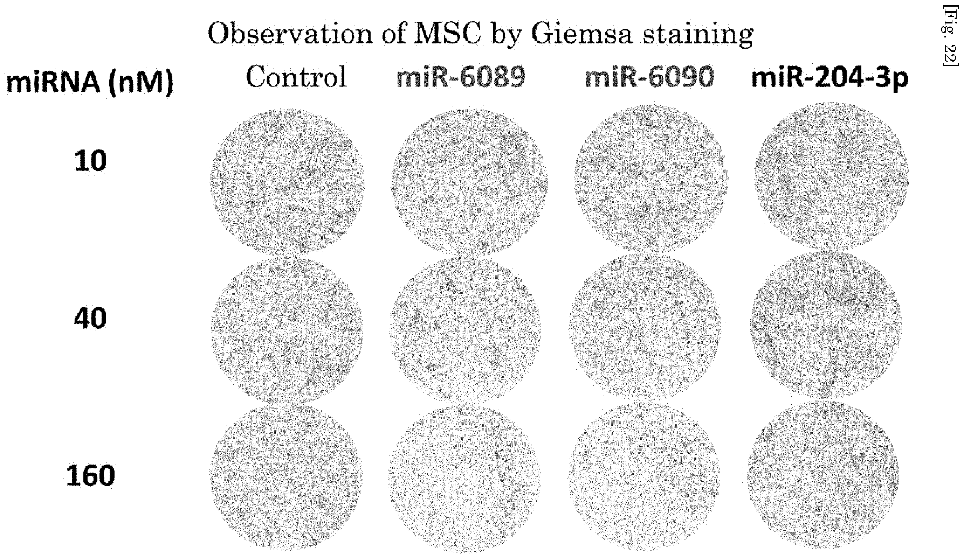

FIG. 22 is photographs illustrating results of investigating the effects of a variety of miRNA contained in exosomes released from human T cells on MSC. In the vertical direction, amounts of added miRNA, and in the horizontal direction, types of added miRNA are aligned. miRNA was not added to controls. Among 40 types of miRNAs, cytotoxicity to MSC was observed in the following two types: miR-6089 and miR-6090. miR-204-3p is illustrated as the one not having cytotoxicity to MSC.

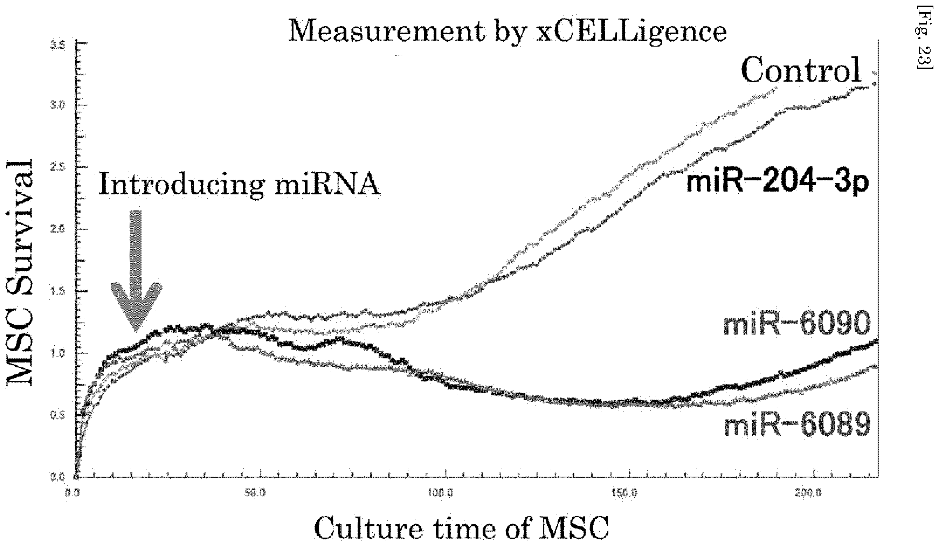

FIG. 23 a graph illustrating results of investigating the effects of each miRNA on survival of MSC. miRNA was added with the final concentration of 40 nM.

### [DESCRIPTION OF THE EMBODIMENTS]

Next, embodiments of the present invention will be explained with reference to drawings and tables. The scope of the present invention is not limited to these embodiments, and a variety of other embodiments will be possible without departing from outline of the present invention.

The therapeutic agent according to the present invention includes extracellular vesicles (exosomes) released from cytotoxic T cells as an active ingredient. The cytotoxic T cells may be derived from humans, monkeys, mice, rats, cattle, horses, camels, sheep, or birds (including chickens and ostriches). Note that the cytotoxic T cells used for treatments are not necessarily derived from the same species; however, cytotoxic T cells derived from the same species are preferably used. In addition, even in cases where cytotoxic T cells derived from the same species are used, the cytotoxic T cells are not necessarily limited to those derived from the subject to be treated (including humans, and animals other than humans), and the therapeutic agent may be extracted from cytotoxic T cells of other individuals.

Although the therapeutic agent includes extracellular vesicles (exosomes) released from the cytotoxic T cells, which are at least 1, or 2 or more of CD4⁺, CD8⁺, CD9⁺, CD63⁺, and TCR⁺ T cells, the cytotoxic T cells are not limited thereto.

The cell-proliferative disease refers to a disease having characteristics in which cells are abnormally proliferated beyond the normal range, and examples thereof include malignant tumors (cancers), and precancerous conditions (conditions where risks of malignant tumors are significantly increased, or precancerous lesions with morphological alterations that easily induce malignant tumors compared to normal tissues).

Furthermore, the therapeutic agent is effective for not only suppressions of cell proliferation, but also suppressions of metastases. In addition, although the effective targets of the therapeutic agent are preferably tumors from precancerous conditions to cancerous conditions, the therapeutic agent is effective for suppressions of proliferation and metastases of any tumors regardless of whether they are benign or malignant.

A method for administrating therapeutic agent of the present invention include a direct injection to tumors, an injection to mesenchymal cells around tumors, an intravenously injection, and a subcutaneous injection, but not limited thereto.

Moreover, the therapeutic agent according to the present invention includes miRNA derived from extracellular vesicles (exosomes) that was released from cytotoxic T cells, as an active ingredient. The miRNA preferably has activities effective in suppressing cell proliferation.

miRNA (micro RNA) is one of ncRNA (non-coding RNA). The miRNA can be found inside cells, and is short RNA having a length of about 20 to 25 bases. The miRNA is thought to have functions to regulate gene expressions.

Such miRNA can be used as being encapsulated in endoplasmic reticulum (exosomes, including artificial liposomes).

In addition, examples of the additives listed in the sixth aspect of the present invention, that is, microbicides, tonicity agents, pH control agents, stabilizing agents, thickening agents, preservatives, fragrances, adhesives, and immune enhancement agents, are described below. Specific examples of the microbicides include iodine preparations and alcohols. Pronase is a specific example of mucosa removing agents. Specific examples of the tonicity agents include sodium chloride and glycerin. Specific examples of the pH control agents include citric acid, gluconic acid, succinic acid, potassium carbonate, and lactic acid. Specific examples of the stabilizing agents and the thickening agents include carrageenan, sodium carboxymethyl cellulose, xanthan gum, guar gum, and pectin. Specific examples of the preservative (preservation agents) include benzoic acid. Specific examples of the adhesives include gelatin, starch, and casein. Specific examples of the immune enhancement agents include agonists of Toll-like receptors such as CpG oligo DNA and poly IC RNA; chemotherapeutics such as taxane compounds; and signaling inhibitors. These examples do not indicate limitations, and any substances can be used as long as they are safe to living cells.

### <Testing Method>

### 1. Mice and Tumor Cell Line

BALB/c (CD90.2) and C57BL/6 (B6) female mice of 6-8 week old were purchased from Japan SLC. CD90.1 congenic BALB/c mice, mutated ERK2 (mERK2) 136-144 (SEQ ID 1: QYIHSANVL) specific H-2Kd-restricted TCR (Vβ10.1/Jβ48 and Vβ8.3/Dβ2.1/Jβ2.6) gene-introduced DUC18 mice (Non Patent Document 20) and CD90.1 congenic DUC18 mice were maintained in the laboratory animal facility of Mie University. CMS5a, CMS7, CT26, 4T1, B16, and B16F10 tumor cell lines were passaged in the D-MEM medium containing 10% FCS. CD8⁺ T cells obtained by culturing DUC18 mouse splenocytes specifically lysed mREK2⁺ CMS5a, but did not lyse mREK2⁻ CMS7, CT26 (to BALB/c background), B16 or B16F10 melanoma (to B6 background). The experimental protocol was evaluated in the Animal Care and Use Committee of Mie University.

### 2. Preparation of Extracellular Microvesicle (ECV) from Culture Medium

FCS was ultracentrifuged at 100,000×g for 4 hours, and then filtrated (0.45 µm and 0.22 µm) to prepare FCS (ECV-free FCS) not containing ECV. Splenocytes prepared from DUC18 mice or CD90.1 DUC18 mice (2×10⁷/ml) were cultured in RPMI-1640 medium containing 10% ECV-free FCS and 1 µg/ml mERK2 peptide. Splenocytes prepared from B6 mice (2×10⁷/ml) were cultured in RPMI-1640 medium containing 10% ECV-free FCS, 1 µg/ml TRP-2 (SEQ ID 2: SVYDFFVWL) and 1 µg/ml gp100 (SEQ ID 3: EGSRNQDWL) peptides (Non Patent Document 21). Splenocytes obtained from BALB/c mice, CMS5a cancer-bearing BALB/c mice, or CD8⁺ T cell-depleted BALB/c mice (2×10⁷ cells/ml) were cultured in 12-well plates on which an anti-CD3 monoclonal antibody (2C11: 2 µg/ml: Biolegend) was solidified, with RPMI-1640 medium containing 10% ECV-free FCS and 1 µg/ml anti-CD28 monoclonal antibody (37.51: eBioscience).

Human peripheral blood monocytes (hPBMC) prepared by using Ficoll-Paque PLUS (manufactured by GE healthcare) gradient were cultured in 12-well plates on which an OKT3 monoclonal antibody (2 µg/ml: Biolegend) was solidified, with RPMI-1640 medium containing 10% ECV-free FCS and 1 µg/ml of CD28 monoclonal antibody (Biolegend). CD8⁺ T cell-depleted BALB/c mice were prepared by intravenously injecting a Lyt-2.2-specific monoclonal antibody (400 µg/mouse) so that the number of CD4⁺ T cells is increased in vitro. On day 4 after starting the culture, each medium was changed to RPMI-1640 medium containing 10% ECV-free FCS and a recombinant IL-2 (rIL-2) (100 IU/ml), and further cultured for 3 days. The obtained supernatants were used as ECV sources. The obtained cells were subjected to flow cytometry analyses using a mouse CD4 (GK1.5) specific monoclonal antibody, a CD8 (53-6.7) specific monoclonal antibody, a TCRVβ (H57-597) specific monoclonal antibody, and a Vβ8.3 (8C1)specific monoclonal antibody, or a human CD4 (OKT4)specific monoclonal antibody, a CD8 (RPA-T8) specific monoclonal antibody, and a TCR (IP26) specific monoclonal antibody (all were obtained from Biolegend) and cytotoxicity assays using carboxyfluorescein succinimidyl ester (CFSE).

ECV was purified according to a protocol using ultracentrifugation. A culture supernatant (about 500 ml) was centrifuged at 10,000×g for 40 minutes, filtrated with 0.45 µm and 0.22 µm filters, and concentrated to 100 ml by ultrafiltration (Kvick Lab Packet 50 KD: GE Healthcare). The concentrated supernatant was filtrated with a 0.22 µm filter, and ultracentrifuged at 120,000×g for 90 minutes (SW28 rotor: Beckman Courter). The obtained ECV precipitation was suspended in 30 ml of PBS, and washed by ultracentrifugation at 120,000×g. Finally, the ECV precipitation was suspended in 1 ml to 2 ml of PBS, and stored at 4°C.

The protein concentration of the obtained purified ECV was measured by using the bicinchoninic acid (BCA) protein assay kit (Pierce). The average number and the average diameter of the purified ECV were measured by the nano tracking assay (LM10-HS: Nanosight). In order to analyze ECV surface proteins by flow cytometry, ECV was immobilized on latex beads, and stained with a fluorescein isothiocyanate (FITC) or phycoerythrin (PE)-conjugated anti-CD4 monoclonal antibody, anti-CD8 monoclonal antibody, anti-CD9 monoclonal antibody (MZ3), anti-CD63 monoclonal antibody (NVG-2), and anti-Vβ8.3 monoclonal antibody (all were obtained from Biolegend). In 0.1 M 2-morpholino ethanesulfonic acid (MES) buffer, 10 µm polystyrene latex beads were mixed with an ECV sample so that an ECV/latex ratio becomes 3. The mixture was shaken on a rotatory shaker at room temperature for 2 hours, and then blocked with 400 mM glycine. The obtained ECV bound to latex was washed 2 times with 2% ECV-free FCS-containing PBS, and stained with monoclonal antibodies.

In order to evaluate kinetics of ECV in vivo and in vitro, 100 µg to 300 µg ECV was stained with 10 µM SYTO RNASelect Green Fluorescent Cell Stain (Molecular Probes) at 37°C for 20 minutes, and unbound staining substances were removed by using the Sephadex G25 Spin Column.

### 3. Differentiation Culture of Bone Marrow Mesenchymal Stem Cell (BM-MSC)

BM-MSC was prepared from femora according to instructions of the attached document (StemCell Technologies Inc.). Both ends of 10 femora obtained from BALB/c or CD90.1⁺ BALB/c were cut, and placed in a mortar with 5 ml of 1% BSA-containing PBS. In the mortar, the femora were softly ground for 5 minutes to be crushed. Red spinal cord cells found were discarded. The red spinal cord cells were removed from crushed femora 5 times as the 1% BSA-containing PBS was changed to fresh one, white pieces of the femora were collected, and incubated with 0.2% Collagenase type I (Sigma)-containing PBS. The femora were vigorously shaken in a water bath at 37°C for 40 minutes, a supernatant containing MSC was filtrated through a 70 µm filter. After 3 times of washing, MSC adhered on a culture plate was cultured in the 20% mouse MSC stimulant-containing MesenCult MSC basic medium (StemCell Technologie Inc.) for 30 days. During the culture, a half volume of the medium was changed with flesh one every 3 days. In order to confirm the ability of differentiation of the obtained MSC into adipocytes and osteocytes, 70% confluent MSC was cultured in the MesenCult MSC basic medium containing 20% adipocyte-forming and osteocyte-forming stimulants for 2 weeks. The cells were stained with Oil Red O (Sigma-Aldrich) for adipocytes, and Alizarin Red S (Wako Pure Chemical Industries, Ltd.) and hematoxylin (Muto Pure Chemicals Co., Ltd.) for osteocytes. Early MSC colonies obtained with the MSC stimulant-containing medium were subjected to Giemsa staining (Wako Pure Chemical Industries, Ltd.). The MSC was stained with a PE-conjugated anti-CD140a monoclonal antibody and an FITC-conjugated anti-Sca-1 monoclonal antibody, and analyzed by flow cytometry (FACScant II. BD) to confirm the purity of the cultured BM-MSC. The cultured MSC was further related to presence of CD29, CD90.1, and CD105, and absence of CD14, CD34, and CD45. The MSC was stained with monoclonal antibodies against each of the molecules, and analyzed by flow cytometry for evaluations.

### 4. Generation of MB-MSC Chimera Mouse

Femora of BALB/c mice (CD90.2) were washed with PBS to prepare bone marrow cells. After culturing, and before transferring of the BM-MSC, 6-Gy radiation was given to BALB/c mice. Cultured MSC obtained from CD90.1⁺ BALB/c mice (1×10⁶/mouse) was mixed with bone marrow cells obtained from femora of BALB/c (5×10⁶/mouse), and the mixed cells were intravenously injected to the irradiated BALB/c mice. The obtained chimera mice were maintained for 2 weeks with 1 mg/ml neomycin (Calbiochem)-containing autoclaved water and feed irradiated with X-rays. On day 60 after MSC transfer, CD90.1⁺ BM-MSC chimera BALB/c mice were used to confirm tumoral MSC.

### 5. Processing of CD8⁺T Cell and ECV Released from CD8⁺ T Cell In Vivo

In order to study the relationship between ECV released from tumor invading CD8⁺ T cells and structural alterations of tumor-associated stroma, CD90.1 DUC18 CD8⁺ T cells on day 7 of culture (1×10⁷/mouse) alone or CD90.1 DUC18 CD8⁺ T cells treated with GW4869 (an ECV-release inhibitor) (1×10⁷/mouse) were intravenously injected to CMS5a cancer-bearing BALB/c mice or BALB/c nude mice, which was on day 10 after subcutaneous inoculation of CMS5a tumor cells (the tumor diameter was about 10 mm). At the same time, an anti-mouse glucocorticoid-induced TNF receptor-related protein (GITR) monoclonal antibody (DTA-1) (2 µg/tumor) was intratumorally injected (inter tumor: i.t.). As explained in Non Patent Document 18, DTA-1 was used to increase accumulation of CD8⁺T cells in the tumor site. Twenty µg/ml of GW4869 was added 24 hours before termination of the culture. On day 1, day 2, day 3, day 5 and day 7 after injection of the cultured CD90.1 DUC18 CD8⁺ T cells, the CMS5a tumor tissues were collected, and subjected to immunohistochemical staining.

CMS5a cells and B16 cells (1×10⁶/mouse) were subcutaneously injected to back skin of each of BALB/c and B6 mice. In week 2 after the injection, mice having 1.2-cm to 1.5-cm diameters of the tumors were selected for ECV processing. ECV obtained from each culture supernatant was intratumorally injected to CMS5a or B16 with a protein amount of 1, 5, or 10 µg, and the tumor diameter after the injection was measured. In addition, on day 3 and day 5 after ECV injection, the tumor was cut with scissors, and then incubated in 0.5% Trypsin and 1 mM EDTA-containing PBS, at 37°C for 60 minutes. The obtained tumor cell suspension was applied to a wool column, washed 3 times with 1% FCS-containing PBS, and subjected to flow cytometry analysis and a test for confirming spheroid formation. For the flow cytometry analysis, an Sca-1, I-Ad, CD11b CD11c, CD73 or CD206 specific FITC-conjugated monoclonal antibody; and an F4/80, Gr-1, or CD140a specific PE-conjugated monoclonal antibody were used. For the test for confirming spheroid formation, cells were cultured in 10% FCS-containing RPMI-1640, with the concentration of 1×10⁵/ml.

To CD90.1⁺ BM-MSC chimera BALB/c mice in which CMS5a was subcutaneously transplanted, 5 µg ((ECV protein mass)/tumor) of DUC18 ECV released from CD8⁺ T cells was intratumorally injected in week 2 after inoculation of the tumor cells. The tumor cell suspensions obtained on day 3 after ECV injection were stained with an FITC-conjugated CD90.1 specific monoclonal antibody, a PE-conjugated CD140a specific monoclonal antibody, and an allophycocyanin (APC)-conjugated Sca-1 specific monoclonal antibody. After removal of 7-amino actinomycin D (7-ADD)-stained cells, the remaining cells were analyzed by flow cytometry.

B16F10 was subcutaneously inoculated, and 50 µg of DUC18 CD8⁺ T cell ECV, CMS5a cancer-bearing BALB/c splenocyte ECV, or BALB/c splenocyte ECV was intratumorally injected on day 7, day 10, and day 13 after inoculation. On day 16after tumor cell inoculation, the tumor derived from B16F10 (having a diameter of about 2 cm) was carefully cut with scissors to observe the tumor invasion. After that, the skin was sutured with a surgical suture. On day 45 after inoculation of the tumor cells, whether lung metastasis of B16F10 exists was observed.

### 6. Processing of ECV Released from CD8⁺ T Cell In Vitro

ECV obtained from cultured splenocytes of DUC18, CMS5a cancer-bearing BALB/c, B16 cancer-bearing B6, BALB/c, and B6 were added to a culture medium of 5×10⁴/ml of CMS5a, B16, CT26, or BM-MSC. The 10% FCS-containing RPMI-1640 medium or the BM-MSC medium (20% MSC stimulant-containing MesenCult MSC basic medium) was used for culturing each of the cells. Furthermore, ECV was added to a mixed culture medium of 5×10⁴/ml of CMS5a, CT26, or B16 cells, and 5×10⁴/ml BM-MSC (cultured in the 10% FCS-containing RPMI-1640 medium), with a concentration of 1 µg or 5 µg (ECV protein)/ml.

On day 4 after starting the culture, the obtained cells were subjected to a test for confirming spheroid formation, and flow cytometry analyses for confirming the total number of the cells and expressions of CD140a and/or Sca-1.

### 7. Fluorescent Immunoassay

The frozen specimens of CMS5a and B16F10 tumors embedded in an OCT compound (Sakura Finetek) were sectioned to a thickness of 3 µm. The tissue sections were air-dried for 2 hours, fixed for 15 minutes in ice-cold acetone, and subjected to immunohistochemistry. The tissue sections were washed 3 times with PBS, and were incubated in a blocking solution (1% BSA, 5% Blocking One Histo (Nacalai Tesque)-containing PBS, 0.2 µg/ml of an anti-mouse CD16/CD32 monoclonal antibody (Biolegend)) at 4°C for 30 minutes. Furthermore, tumor sections on glass slides were double labelled with a PE-conjugated monoclonal antibody and an FITC-conjugated monoclonal antibody dissolved in 1% BSA and 5% Blocking One Histo-containing PBS in a humidified chamber at room temperature for 1 hour. The glass slides were washed 3 times with 0.02% Tween-20-containing PBS, and treated with DAPI-containing ProLong Gold Antifade Mountant (Invitrogen-Life Technologies), and observed under a fluorescence microscope (BX53F, manufactured by Olympus). The pictures of the tissues stained with PE, FITC, and DAPI were superimposed by using Photoshop elements software (Adobe Systems). For the fluorescent immunoassay, PE-conjugated monoclonal antibodies against CD8, CD140a, Ki-67, CD31, CD11b, ER-TR7, and TGF-β1; and FITC-conjugated monoclonal antibodies against ER-TR7, Sca-1, F4/80, Gr-1, CD90.1, and α-smooth muscle actin (α-SMA) were used.

### 8. Cytotoxicity Assay

CMS5a, CMS7, and CT26 cells were labelled with 2.5 mM carboxyfluorescein diacetate succinimidyl ester (CFSE) at 37°C for 6 minutes. Cells were washed 3 times with 10% FCS-containing RPMI-1640, and CFSE-labelled CMS5a was used as a target cell. mERK2 peptide-stimulated DUC18 splenocytes were mixed with CFSE-labelled CMS5a, CMS7, or B16 cells (1×10⁵) in a 24 well plate at a ratio of 1, 5, and 10. After 12 hours of incubation, the remaining cells were analyzed by flow cytometry. For each sample, 20,000 of cells not labelled with CFSE were collected, and viable cells labelled with CFSE were counted. The viability was determined as an average value of 2 wells, and % cellular cytotoxicity was calculated according to the document (Non Patent Document 18).

### 9. Analysis of miRNA in ECV Vesicle

By using the 3D Gene Microassay System (Toray Industries, Inc.), 100 µg of ECV released from CD8⁺ T cells obtained from cultured DUC18 (2 lots), CMS5a cancer-bearing BALB/c, and BALB/c splenocytes; and 100 µg of ECV released from CD4⁺ T cells obtained from CD8⁺ T cell-depleted BALB/c mouse splenocytes were analyzed to identify miRNA. For normalized raw data of the microarray, samples were compared each other. The 3 miRNA were identified as dominant in ECV of DUC18, compared to ECV of CMS5a cancer-bearing BALB/c and of CD4⁺ BALB/c, and were subjected to a functional analysis.

According to RNA sequences of the miRBase, mouse miR298-5p (SEQ ID 4: GGC AGA GGA GGG CUG UUC UUC CC), miR-298-3p (SEQ ID 5: GAG GAA CUA GCC UUC UCU CAG C), miR1943-5p (SEQ ID 6: AAG GGA GGA UCU GGG CAC CUG GA), miR-1943-3p (SEQ ID 7: CAG GUG CCA GCU CCU CCC UUC), miR-5099-5p (SEQ ID 8: GUU AGA AAU UAC AUU GAU UUA A), miR5099-3p (SEQ ID 9: UUA GAU CGA UGU GGU GCU CC), miR-150-5p (SEQ ID 10: UCU CCC AAC CCU UGU ACC AGU G), miR-150-3p (SEQ ID 11: CUG GUA CAG GCC UGG GGG AUA G), miR-223-5p (SEQ ID 12: CGU GUA UUU GAC AAG CUG AGU UG), miR-223-3p (SEQ ID 13: UGU CAG UUU GUC AAA UAC CCC A), miR-3470b-5p (SEQ ID 14: UCA CUC UGU AGA CCA GGC UGG), and miR-3470b-3p (SEQ ID 15: CCU GCC UCU GCC UCC CGA) were synthesized, and annealed between 5p and 3p (Hokkaido system Science Co., Ltd.).

ECV obtained from human PBMC was used as a temporal vesicle for the functional analysis of synthesized miRNA. In a 12 well plate, an anti-CD3 and anti-CD28 monoclonal antibodies were added to RPMI-1640 medium containing 10% FCS and 100 IU/ml rIL-2, and the human PBMC was cultured therein and stimulated for 3 days. The cells were washed 2 times with RPMI-1640 medium not containing FCS, and stimulated human PBMC (1×10⁸) was suspended in 1.5 ml of 1% DMSO-containing RPMI-1640 medium. After mixing the suspension with an RNA pool of miR-298 (50 µg), miR-1943 (50 µg), and miR-5099 (50 µg); or of miR-150 (50 µg), miR-223 (50 µg), and miR-3470b (50 µg), and the mixture was subjected to electroporation. The obtained cells were cultured in 10% FCS-containing RPMI-1640 medium not containing ECV for 20 hours, and supernatants were filtrated through 0.45 and 0.22 filters, and then ultracentrifuged (120,000 g) to obtain synthesized miRNA-containing ECV.

### 10. Exploration of MSC Cytotoxic miRNA

Monocytes (PBMC) were separated from human peripheral blood by using Ficoll. The PBMC (2×10⁵ cells/ml) was cultured in GT-T503 medium (TAKARA Bio Inc.) supplemented with 0.6% self-plasma, 0.2% human serum albumin (CSL Behring), and 600 IU/ml rIL-2, for 2 weeks. Culture plates coated with a 5 µg/ml OKT3 antibody (BioLegend) and 25 µg/ml RetroNectin (TAKARA Bio Inc.) were used. The cell populations after the culture were analyzed for presence or absence of CD4 and CD8 by flow cytometry. Furthermore, culture supernatants after the culture were centrifuged at 10,000 g for 20 min, and filtrated through 0.45 µm and 0.22 µm filters to remove cell debris and aggregated proteins. In addition, the filtrate was ultracentrifuged at 120,000 g for 70 min to separate exosomes released from cultured human T cells.

The diameters of the obtained exosomes were measured by Nano-Tracking Analysis (NTA). Furthermore, flow cytometry analyses (BD: FACSCant) were conducted by using a variety of antibodies electrostatically bound to latex beads (4 µm diameter: Life Technologies) to investigate surface molecules on T cells and exosomes.

miRNA contained in exosomes released from cultured human T cells was analyzed by microarray (Toray: 3D-Gene). The specified 40 kinds of miRNA were synthesized one by one from more to less of abundance. Synthesized miRNA were added to cultured mesenchymal stem cells (MSCs) derived from human adipose tissues, and cellular cytotoxicity was investigated. Cellular cytotoxicity was investigated by methods of the following: (1) a method in which synthesized miRNA is added to cultured MSC, and then the cultured MSC is subjected to Giemsa staining (Wako), and (2) a method in which a variety of miRNA is added to MSC cultured in a dedicated plate, and then viability of the MSC is measured by using xCELLigence (ACEA BioSciences) that is equipment to measure cell viability as electrical resistance values.

### 11. Statistical Analysis

Data of 2 groups were analyzed by Mann-Whitney U test. Equality of variance was confirmed by Levine's test, and comparison of data between 2 groups was analyzed by Student's t test. Statistical significance was determined with p<0.05. For statistical calculations, SPSS statistical software v21.0 (IBM) was used.

### <Test Result>

### 1. Suppression of Tumor Proliferation by ECV Released from CD8⁺ T Cell

First, the inventors of the present invention examined influence of ECV derived from TCR-stimulated lymphocytes on tumor proliferation. Mutated ERK2 peptide-stimulated splenocytes of TCR gene transgenic DUC18 mice; CMS5a cancer-bearing BALB/c mouse, BALB/c mouse, CD8⁺ T cell deficient BALB/c mouse splenocytes, or hPBMC stimulated with both a CD3 specific monoclonal antibody and a CD28 specific monoclonal antibody were cultured for 4 days, and then further cultured for 3 days under the presence of rIL-2 (100 IU/ml). All of the splenocytes of cultured DUC18, CMS5a cancer-bearing BALB/c, and BALB/c showed the phenotype of CD4-CD8⁺ on day 4 of the culture. Within the splenocytes of CD8⁺ T cell deficient BALB/c mice, 65% were CD4⁺ and CD8⁻. Within the cultured hPBMC, CD8 was 70%, and CD4 was 30% (FIG. 1A).

Furthermore, cultured CD8⁺ DUC18 splenocytes showed cytotoxicity against mERK2⁺ CMS5a, but did not show toxicity against mERK2- CMS7 or CT26, and mERK2 peptide-stimulated CMS7 was lysed (FIG. 2). The obtained supernatants were ultracentrifuged, and ECV was purified from each of the cells (DUC18, CMS5aTB, BALB/c, CD4 BALB/c, and hPBMC). All types of ECV were existed in the supernatants with 0.6 µg/ml to 1.0 µg/ml of protein concentrations, at about 4×10⁹ to 8×10⁹/ml, and 110 nm to 140 nm of average diameters (FIGs. 3A and 3B). Surface markers of DUC18 ECV were investigated. In accordance with phenotypes of parental cells, DUC18 ECV expressed CD8, TCRVβ8.3, and CD63 in low levels, and highly expressed CD9, which has already been known as an ECV marker (FIG. 1B).

To BABL/c mice in which CMS5a was subcutaneously inoculated, DUC18 ECV, CMS5aTB ECV, and BALB/c ECV were intratumorally (having a tumor diameter of 1.2 cm to 1.5 cm) injected. Surprisingly, proliferation of CMS5a treated with DUC18 ECV and BALB/c ECV was terminated and significantly decreased respectively, compared to a CMS5aTB ECV-treated or an untreated group (FIG. 1C). Moreover, spheroid formation after 1 day culture of CMS5a suspensions was not observed in a DUC18 ECV-treated group, but observed in an untreated group or a CMS5a TB ECV-treated group (FIGs. 1C and 1D). Similarly, in a CMS5a cancer-bearing group and a CT26 cancer-bearing group of BALB/c nude mice, intratumoral injection of DUC18 ECV decreased the tumor. In addition, when CD4 BALB/c ECV was intratumorally injected into CMS5a tumors, suppression of proliferation was not observed (FIGs. 1C and 4). In CMS5a tumor to which DUC18 ECV and BALB/c ECV were injected, decrease in Ki-67 expression was observed immunohistochemically (FIG. 1E). As a conclusion of these findings, except under the tumor environments, activated CD8⁺ T cells release ECV that suppresses tumor proliferation as cellular immunity independently and nonspecifically.

### 2. Decrease in CD140a Expression on Tumor by ECV Released from CD8⁺ T Cells

Activated ECV released from CD8⁺ T cells may directly suppress increase in tumor cells, or may suppress tumor proliferation through acting to tumor-bearing cells. In order to solve this question, the inventors of the present invention have investigated change of tumor invading cell populations that have been known to regulate tumor proliferation. On day 3 after injection of DUC18 ECV and BABL/c ECV to CMS5a tumor, the proportions of F4/80⁺ CD206⁺ macrophages or F4/80⁺ I-A^{d+} macrophages, and CD11c⁺ dendritic cells CD11b⁺Gr-1⁺MDSC or CD4⁺ and CD8⁺ lymphocytes were not changed (FIG.5), the numbers of proliferating tumor cells, CD140⁺ mesenchymal marker positive cells including BM-MSC and/or CAF cells were largely decreased by injection of DUC18 ECV (FIGs. 6A and 6B). These data were confirmed also with the fact that expression of CD140a was decreased in CMS5a to which DUC18 ECV and BALB/c ECV were injected (FIG. 6C). Alternatively, kinetics of ECV expression was confirmed on ECV obtained from culture supernatants of TRP-2 and GP100 peptide-stimulated B6 splenocytes on day 5, day 7, day 10, and day 15. TRP-2 specific CD8⁺ lymphocytes and gp100 specific CD8⁺ lymphocytes were gradually increased in the culture, and they reached 95% and 3% respectively on day 15 (FIG. 7). Interestingly, the peak of decrease in CD140a expression by intratumoral injection was observed on ECV obtained on day 7 of the culture, and ECV released from TRP-2 and gd100 specific CD8⁺ T cells act similarly to unrelated CMS5a and related B16 (FIG. 6D). Decrease in functional ECV production by peptide specific CD8⁺T cells observed on day 15 after injection was thought to be relevant to decay of lymphocytes.

These results demonstrate that ECV released from CD8⁺ T cells affects proliferation and progression of tumors without specificities.

### 3. Mesenchymal Stromal Cell-Mediated Downregulation of Malignant Transformation of Tumor by ECV Released from CD8⁺ T Cell

DUC18 ECV does not directly affect CD140a expression levels not only on CMS5a cells, but also on CD26, 4T1, and B16 cells. In vitro annexin V staining demonstrated that apoptosis cannot be induced to CMS5a, CT26, 4T1, or CMS7, and thus direct suppression effects of ECV released from CD8⁺ T cells to tumor cells was denied (FIG. 8). Moreover, in immunohistochemistry, CMS5a tumor treated with DUC18 ECV demonstrated depletion of BM-MSC (CD140a⁺ Sca-1⁺) and depletion of CAF (ER-TR7⁺ α⁻ smooth muscle actin [SMA]⁺), and decrease in TGF-β1 expression (FIG. 9A). Then, the inventors of the present invention have investigated relationship between BM-MSC, which is representative of mesenchymal tumor-associated stromal cells, and ECV released from CD8⁺ T cells. The number of cultured BM-MCS was dramatically decreased upon addition of DUC18 ECV and B6 ECV on day 3 after the addition (FIG. 10); however, such a reaction was observed on ECV released from human PBMC (FIG. 9B). In addition, when DUC18 ECV was added to a mixture of cultured BM-MSC and tumor cells, CD140a expression of CMS5a and B16 cells; and spheroid formation of CMS5a, 4T1, CT26, and B16 were downregulated under the presence of DUC18 ECV compared to a CMS5aTB ECV-treated group (FIG. 9C). Accordingly, transition of tumor to mesenchyme induced by interaction with BM-MSC was thought to be inhibited due to loss of BM-MSC by ECV released from CD8⁺ T cells. In order to confirm that tumor invading BM-MSC is depleted under the presence of ECV derived from CD8⁺T cells in vivo, DUC18 ECV, BALB/c ECV and hPBMC ECV were intratumorally injected into CMS5a cancer-bearing CD90.1⁺ BM-MCS chimera BALB/c mice (FIG. 11).

CD90.1⁺ cells containing BM-MSC differentiated cells (for example, CAF, cancer-related fibroblasts, pericytes), which were 5% and about 15% of injected CD140a⁺ Sca-1⁺ BM-MSC, were found inside CMS5a tumor of the chimera mice. These tumor invading CD90.1⁺ cells were not affected by hPBMC ECV, while they were depleted by intratumoral injection of DUC18 ECV and BALB/c ECV (FIG. 9D).

ECV will be incorporated in both BM-MSC and tumor cells. B16 cells and CMS5a cells co-cultured with BM-MSC incorporate SYTO RNASelect-labelled DUC18, CMS5a TB, or hPBMC ECV, and intensities of green fluorescence were decreased soon after the incorporation. Tumor cells contacted with BM-MSC seem to decompose ECV-derived miRNA immediately after they incorporate ECV (FIG. 12A). According to RNA microarray analysis (3D Gene, manufactured by Toray), it was demonstrated that lysozyme mRNA was largely increased in tumor cells contacted with BM-MSC (FIG. 13). Flow cytometry analysis and immunohistochemistry analysis of CMS5a treated with SYTO RNASelect-labelled DUC18 ECV also demonstrated that green fluorescence label was observed within the region of CD140a⁺ or Sca-1⁺ stroma 30 minutes after ECV treatment, but was not observed within the region of cancer (FIGs. 12B and 12C).

### 4. Involvement of Novel miRNA in Inhibition of Tumor Progression via Mesenchymal Stromal Cell

Since hPMBC ECV was unresponded, but self- and allogenic CD8⁺ T cells-derived ECV was responded to killing of BM-MSC, involvement of miRNA contained in ECV can be suggested. Therefore, total RNA derived from DUC18 (2 lots), CMS5a TB, and CD4 Balb/c ECV were analyzed by miRNA microarray (3D Gene, manufactured by Toray). By comparing global normalization values and heat mapping data among miRNA, it was clarified that miR-298, miR-351, miR-700, miR-141, miR-1943, miR-1249, miR-344g, miR-23b, miR-370, miR-1199, miR-5113, miR-5114, miR-6347, miR-6392, and miR-5099 were dominant in DUC18 ECV; and miR-150, miR-223, and miR-3470b were dominant in CMS5a ECV (FIG. 14A). As expected, since self-suppressive effects of tumors have been reported for DUC18 ECV-derived miR-298, miR-141, miR-1249, miR-23b, and miR-370, the selected types of miRNA were confirmed to be right (FIG. 14B and FIG. 15). Furthermore, 3 kinds of miRNA (miR-298, miR-1943, and miR-5099) were expected to be the most effective among the DUC18 ECV. The 5p and 3p oligonucleotides of these 3 kinds of miRNA were synthesized, annealed, and introduced into cultured BM-MSC. When compared to the cases where miR-1943 and miR-5099 were introduced, introduction of miR-298 induced large decrease in BM-MSC (FIG. 14C). When miR-150, miR-223, and miR-3470b, which were dominant in CMD5a TB ECV, were introduced, killing actions to BM-MSC were not observed. From these results, miR-298 was found for the first time as miRNA capable of killing BM-MSC. In terms of similarity of immune systems and basic research of cancer, there exists miRNA having such specific functions among miRNA contained in exosomes obtained from human cytotoxic T cells.

### 5. Prevention of Invasion and Metastasis of Tumor by Treating with ECV Released from CD8⁺ T cell

Invasions and metastases of tumors will be indicators of epithelial to mesenchymal transitions and aggravations of tumors. Therefore, in order to investigate effects to invasions and metastases, the inventors of the present invention intratumorally injected DUC18 ECV, CMS5a TB ECV, or BALB/c ECV to mice, in which B16F10 cells were subcutaneously inoculated, on day 10, day 13, and day 16 after inoculation. On day 18 after injection, the status of tumor invasion was observed, and the B16F10 tumor was surgically cut, and then skin was sutured. In the untreated group, 50% of the tumor, and in the CMS5a ECV treatment group, 33% of the tumor can be removed. In all of the unremovable tumors, invasion to fascia was observed (see Table 1).

**Table 1. Fascia Invasion of B16F10 after treatment of DUC18, BALB/c, and CMS5aTB EMVs at the primary tumor sites.**

| i.t. treatment | Number of mice with tumor invasion/total mice (%) |
|---|---|
| - | 6/12 (50%) |
| DUC18 EMVs | 0/13 (0%) |
| CMS5aTB EMVs | 2/3 (66.7%) |
| BALB/c EMVs | 0/3 (0%) |

| | |
|---|---|
| B16F10-bearing mice shown fascia invasion through tumor capsule (impossible to remove the primary tumors surgically) were counted. | |

Meanwhile, in the DUC18 ECV and BALB/c ECV injected groups, all tumors were removable, and all mice survived on day 45 (FIG. 16A and FIG. 16B). In addition, on day 18 after inoculation of B16F10 cells, decrease in CD140a⁺ Sca⁻1⁺ mesenchymal stroma was shown in the mice intratumorally injected with DUC18 ECV and BALB/c ECV by immunohistological analysis (FIG. 16C). As expected, lung metastasis of B16F10 was largely suppressed in the mice injected with DUC18 ECV or BALB/c ECV compared to the untreated mouse (FIG. 16D). These results demonstrate that ECV released from CD8⁺ T cells suppresses aggravations of tumors.

### 6. Peripheral Circulating Activated CD8⁺ T Cells Intratumorally Invade at Angiogenic Region, and Destroy Tumor-Associated Stromal Structure

Finally, the inventors of the present invention have investigated whether CD8⁺ T cells would invade into tumor, and destroy a tumor-associated stromal structure while releasing ECV. BALB/c or BALB/c nude mice were subcutaneously inoculated with CMS5a. When a diameter of CMS5a became about 1 cm, the cultured CD90.1⁺ DUC18 CD8⁺ T cells untreated or treated with GW4869 (an inhibitor of exosome production) (FIG. 17A) were intratumorally injected. The statuses of tumor invaded CD90.1⁺ DUC18 CD8⁺ T cells and stroma were investigated over time by immunostaining of tumor sections. Regardless of with or without GW4869 treatments, CD90.1⁺ CD8⁺ T cells were found in the Sca-1⁺ CD31⁺ angiogenic region of CMS5a tumor-associated stroma composed of endothelial precursor cells and BM-MSC, 24 hours after injection (FIG. 17B). Surprisingly, the Sca-1⁺ or CD140a⁺ region of CMS5a tumor disappeared on day 3 after transfer of CD90.1⁺ CD8⁺ T cells, and this status persisted until day 7 (FIG. 17C). The effect for destroying stroma was found more in wild type BALB/c mice than BALB/c nude mice. This may be due to intratumoral invasion of BALB/c-derived CD8⁺T cells together with injected CD90.1⁺ CD8⁺ T cells. Furthermore, since disappearance of Sca-1⁺ or CD140a⁺ stroma was not observed in the group injected with GW4869-treated CD90.1⁺ CD8⁺ T cells (FIG. 17C), suggesting that intratumorally invaded CD8⁺ T cells produce ECV to destroy the tumor-associated stromal structure, similarly to the result where purified ECV was used. CD90.1⁺ ECV released from CD8⁺ T cells strongly express CD9 and CD90.1, and hardly express CD8 (FIG. 18D). In the CMS5a tumor 24 hours after injection of CD90.1⁺ CD8⁺ T cells, ECV-derived CD9 and CD90.1 (FIG. 18E) and their fusion signal (FIG. 18F) were found in the CD140a⁺ Sca-1⁺ stromal region, but they were not observed in the tumor of the GW4869-treated CD90.1⁺ CD8⁺ T cell-treated group.

### 7. MSC Cytotoxic miRNA can be Specified in Exosome Released from Cultured Human T cell

T cell populations obtained from monocytes separated from human peripheral blood were cultured for 2 weeks, and the flow cytometry analysis demonstrated that the obtained T cell population was CD8 dominant (FIG. 19). The Nano-Tracking analysis demonstrated that the diameters of exosomes released from these T cells to the culture supernatant were about 150 nm (FIG. 20).

The surface antigens on exosomes and cultured human T cells were analyzed by flow cytometry. Tetraspanin molecules as exosome markers (CD9, CD63, CD81), CD8, and HLA class I molecule were expressed (FIG. 21).

Forty types of miRNA contained in the exosomes were added to culturing MSC. As a result of cellular cytotoxicity tests, 2 types of miRNA (miR-6089 and miR-6090) could be identified (FIG. 22 and FIG. 23).

The MSC cytotoxic miRNA thus obtained can be applied to a therapeutic agent for cell-proliferative disorders.

Since the immune system of human is almost similar to that of mouse, the findings obtained on mouse in vivo and in vitro can be applied to human as they are.

Thus, this embodiment can provide a therapeutic agent associated with suppression of proliferation and metastasis of tumor, the therapeutic agent including exosomes released from cytotoxic T cells and targeting cancer stromal/mesenchymal cells.

The prior art documents related to the present invention will be listed below. Although these documents have not been explained in the specification as referring to their numbers, they will be prior art of the present invention.

### [Prior Art Document]

### [Non Patent Document]

Non Patent Document 1: Houlihan DD, Mabuchi Y, Morikawa S, Niibe K, Araki D, Suzuki S, Okano H, Matsuzaki Y. Isolation of mouse mesenchymal stem cells on the basis of expression of Sca-1 and PDGFR-α. Nat. Protoc. (2012) 7: 2103-2111.
Non Patent Document 2: Vajkoczy P, Blum S, Lamparter M, Mailhammer R, Erber R, Engelhardt B, Vestweber D, Hatzopoulos AK. Multistep nature of microvascular recruitment of ex vivo-expanded embryonic endothelial progenitor cells during tumor angiogenesis. J. Exp. Med. (2003) 197: 1755-1765.
Non Patent Document 3: Joyce JA, Pollard JW. Microenvironmental regulation of metastasis. Nat Rev Cancer. (2009) 9: 239-252.
Non Patent Document 4: Tran E, Chinnasamy D, Yu Z, Morgan RA, Lee CC, Restifo NP, Rosenberg SA. Immune targeting of fibroblast activation protein triggers recognition of multipotent bone marrow stromal cells and cachexia. J Exp Med. (2013) 210: 1125-1135.
Non Patent Document 5: Nieto MA. The ins and outs of the epithelial to mesenchymal transition in health and disease. Annu. Rev. Cell Dev. Biol. (2011) 27:347-376.
Non Patent Document 6: Koh BI, Kang Y. The pro-metastatic role of bone marrow-derived cells: a focus on MSCs and regulatory T cells. EMBO reports. (2012) 13: 412-422.
Non Patent Document 7: Nieto MA1, Cano A. The epithelial-mesenchymal transition under control: global programs to regulate epithelial plasticity. Semin. Cancer Biol. (2012) 22: 361-368.
Non Patent Document 8: Filipazzia P, Burdeka M, Villab A, Rivoltinia L, Huber V. Recent advances on the role of tumor exosomes in immunosuppression and disease progression. Semin. Cancer Biol. (2012) 22: 342-349.
Non Patent Document 9: Ono M, Kosaka N, Tominaga N, Yoshioka Y, Takeshita F, Takahashi RU, Yoshida M, Tsuda H, Tamura K, Ochiya T. Exosomes from bone marrow mesenchymal stem cells contain a microRNA that promotes dormancy in metastatic breast cancer cells. Sci Signal. (2014) 7: ra63.
Non Patent Document 10: Boelens MC, Wu TJ, Nabet BY, Xu B, Qiu Y, Yoon T, Azzam DJ, Twyman-Saint Victor C, Wiemann BZ, Ishwaran H, Ter Brugge PJ, Jonkers J, Slingerland J, Minn AJ. Exosome transfer from stromal to breast cancer cells regulates therapy resistance pathways. Cell. (2014) 159: 499-513.
Non Patent Document 11: Webber J, Steadman R, Mason MD, Tabi Z, Clayton A. Cancer exosomes trigger fibroblast to myofibroblast differentiation. Cancer Res. (2010) 70: 9621-9630.
Non Patent Document 12: Chalmin F, Ladoire S, Mignot G, Vincent J, Bruchard M, Remy-Martin JP, Boireau W, Rouleau A, Simon B, Lanneau D, De Thonel A, Multhoff G, Hamman A, Martin F, Chauffert B, Solary E, Zitvogel L, Garrido C, Ryffel B, Borg C, Apetoh L, Rebe C, Ghiringhelli F. Membrane-associated Hsp72 from tumor-derived exosomes mediates STAT3-dependent immunosuppressive function of mouse and human myeloid-derived suppressor cells. J. Clin. Invest. (2010) 120: 457-471.
Non Patent Document 13: Pucci F, Pittet MJ. Molecular pathways: tumor-derived microvesicles and their interactions with immune cells in vivo. Clin. Cancer Res. (2013) 19: 2598-2604.
Non Patent Document 14: Roccaro AM, Sacco A, Maiso P, Azab AK, Tai YT, Reagan M, Azab F, Flores LM, Campigotto F, Weller E, Anderson KC, Scadden DT, Ghobrial IM. BM mesenchymal stromal cell-derived exosomes facilitate multiple myeloma progression. J Clin Invest. (2013) 123: 1542-1555.
Non Patent Document 15: Shimoda M, Principe S, Jackson HW, Luga V, Fang H, Molyneux SD, Shao YW, Aiken A, Waterhouse PD, Karamboulas C, Hess FM, Ohtsuka T, Okada Y, Ailles L, Ludwig A, Wrana JL, Kislinger T, Khokha R. Loss of the Timp gene family is sufficient for the acquisition of the CAF-like cell state. Nat. Cell Biol. (2014) 16: 889-901.
Non Patent Document 16: Hinrichs CS1, Gattinoni L, Restifo NP. Programming CD8+ T cells for effective immunotherapy. Curr. Opin. Immunol. (2006) 18: 363-370.
Non Patent Document 17: Callahan MK, Wolchok JD. At the bedside: CTLA-4- and PD-1-blocking antibodies in cancer immunotherapy. J. Leukoc. Biol. (2013) 94: 41-53.
Non Patent Document 18: Ishihara M, Seo N, Mitsui J, Muraoka D, Tanaka M, Mineno J, Ikeda H, Shiku H. Systemic CD8+ T cell-mediated tumoricidal effects by intratumoral treatment of oncolytic herpes simplex virus with the agonistic monoclonal antibody for murine glucocorticoid-induced tumor necrosis factor receptor. PLoS One. (2014) 9: e104669.
Non Patent Document 19: Prakash MD, Munoz MA, Jain R, Tong PL, Koskinen A, Regner M, Kleifeld O, Ho B, Olson M, Turner SJ, Mrass P, Weninger W, Bird PI. Granzyme B promotes cytotoxic lymphocyte transmigration via basement membrane remodeling. Immunity. (2014) 41: 960-972.
Non Patent Document 20: Ikeda, H., N. Ohta, K. Furukawa, H. Miyazaki, L. Wang, K. Kuribayashi, L.J. Old, and H. Shiku. 1997. Mutated mitogen-activated protein kinase: a tumor rejection antigen of mouse sarcoma. Proc. Natl. Acad. Sci. USA. 94:6375-6379.
Non Patent Document 21: Ly LV, Sluijter M, van der Burg SH, Jager MJ, van Hall T. Effective cooperation of monoclonal antibody and peptide vaccine for the treatment of mouse melanoma. J Immunol. (2013) 190: 489-496.
Non Patent Document 22: Griffin MD1, Ritter T, Mahon BP. Immunological aspects of allogeneic mesenchymal stem cell therapies. Hum Gene Ther. 2010 Dec;21(12):1641-55.
Non Patent Document 23: Bexell D, Gunnarsson S, Tormin A, Darabi A, Gisselsson D, Roybon L, Scheding S, Bengzon J. Bone marrow multipotent mesenchymal stroma cells act as pericyte-like migratory vehicles in experimental gliomas. Mol Ther. (2009) 17: 183-190.
Non Patent Document 24: Eikawa S, Mizukami S, Udono H. Monitoring multifunctionality of immune-exhausted CD8 T cells in cancer patients. Methods Mol Biol. (2014) 1142: 11-17.
Non Patent Document 25: Shimada M, Yoshizaki S, Ichino M, Klinman DM, Okuda K. Apoptosis of antigen-specific CTLs contributes to low immune response in gut-associated lymphoid tissue post vaccination. Vaccine. (2014) 32: 5198-5205.
Non Patent Document 26: Arina A, Schreiber K, Binder DC, Karrison TG, Liu RB, Schreiber H. Adoptively transferred immune T cells eradicate established tumors despite cancer-induced immune suppression. J Immunol. (2014) 192: 1286-1293.
Non Patent Document 27: Maciag PC, Seavey MM, Pan ZK, Ferrone S, Paterson Y. Cancer immunotherapy targeting the high molecular weight melanoma-associated antigen protein results in a broad antitumor response and reduction of pericytes in the tumor vasculature. Cancer Res. (2008) 68: 8066-8075.
Non Patent Document 28: Ochs K, Sahm F, Opitz CA, Lanz TV, Oezen I, Couraud PO, von Deimling A, Wick W, Platten M. Immature mesenchymal stem cell-like pericytes as mediators of immunosuppression in human malignant glioma. J Neuroimmunol. (2013) 265: 106-116.
Non Patent Document 29: Mi Z, Bhattacharya SD, Kim VM, Guo H, Talbot LJ, Kuo PC. Osteopontin promotes CCL5-mesenchymal stromal cell-mediated breast cancer metastasis. Carcinogenesis. (2011) 32: 477-487.
Non Patent Document 30: McDonald DM, Baluk P. Significance of Blood Vessel Leakiness in Cancer Cancer Res. (2002) 62: 5381-5385.
Non Patent Document 31: Yoong KF, McNab G, Hubscher SG, Adams DH. Vascular adhesion protein-1 and ICAM-1 support the adhesion of tumor-infiltrating lymphocytes to tumor endothelium in human hepatocellular carcinoma. J Immunol. (1998) 160: 3978-3988.
Non Patent Document 32: Nandi A1, Estess P, Siegelman M. Bimolecular complex between rolling and firm adhesion receptors required for cell arrest; CD44 association with VLA-4 in T cell extravasation. Immunity. (2004) 20: 455-465.
Non Patent Document 33: Ding Z, Xiong K, Issekutz TB. Chemokines stimulate human T lymphocyte transendothelial migration to utilize VLA-4 in addition to LFA-1. J Leukoc Biol. (2001) 69: 458-466.
Non Patent Document 34: Stauss HJ, Morris EC. Immunotherapy with gene-modified T cells: limiting side effects provides new challenges. Gene Ther. (2013) 20: 1029-1032.
Non Patent Document 35: Winograd R, Byrne K, Evans RA, Odorizzi PM, Meyer AR, Bajor DL, Clendenin C, Stanger BZ, Further EF, Wherry EJ, Vonderheide RH. Induction of T cell immunity overcomes complete resistance to PD-1 and CTLA-4 blockade and improves survival in pancreatic carcinoma. Cancer Immunol Res. (2015) Feb 12. pii: canimm.0215.2014.

## Claims

1. A therapeutic agent for cell-proliferative diseases, the therapeutic agent comprising extracellular vesicles (exosomes) released from cytotoxic T cells.

2. The therapeutic agent for cell-proliferative diseases according to claim 1, wherein the cytotoxic T cells comprise extracellular vesicles (exosomes) released from at least one of human CD4⁺, CD8⁺, CD9⁺, CD63⁺, and TCR⁺ T cells.

3. The therapeutic agent for cell-proliferative diseases according to claim 2, wherein the cytotoxic T cells comprise extracellular vesicles (exosomes) released from CD8⁺ T cells.

4. The therapeutic agent for cell-proliferative diseases according to claim 2 or 3, wherein the extracellular vesicles (exosomes) comprise miRNA effective in suppressing cell proliferation.

5. The therapeutic agent for cell-proliferative diseases according to claim 4, the therapeutic agent comprising the miRNA effective in suppressing cell proliferation.

6. The therapeutic agent for cell-proliferative diseases according to claim 2, the therapeutic agent further comprising one or more selected from microbicides, mucosa removing agents, tonicity agents, pH control agents, stabilizing agents, thickening agents, preservatives, adhesives, and immune enhancement agents.

7. The therapeutic agent for cell-proliferative diseases according to claim 2, wherein the therapeutic agent is injected to tumor tissues, to mesenchymal cells residing inside tumor tissues, intravenously or subcutaneously.

8. A method of extracting miRNA for treating cell-proliferative diseases, the method comprising collecting exosomes released from cytotoxic T cells, and identifying miRNA effective in suppressing cell proliferation in the exosomes.

9. A therapeutic agent for cell-proliferative diseases, the therapeutic agent comprising miRNA effective in suppressing cell proliferation, the miRNA being obtained by the method according to claim 8.

10. A method of identifying MSC cytotoxic miRNA, the method comprising adding miRNA having a base sequence that is same as a base sequence of miRNA identified by the method according to claim 8 to cultured human mesenchymal stem cells (MSCs), culturing the MSC, and investigating toxic activity to the MSC to evaluate MSC cytotoxicity of the miRNA.

11. A therapeutic agent for cell-proliferative disorders, the therapeutic agent comprising MSC cytotoxic miRNA identified by the method according to claim 10.


SEQUENCE LISTING

<110>  MIE University
       KYOTO University

<120>  CD8+ T Cells Play as a Destroyer of Tumor Stromal Cells by
       Missile Attacking in addition to the Conventional Contact
       Cytolysis of Tumor Cells

<130>  JP2016002MIK

<150>  JP2015-058467
<151>  2015-03-20

<160>  15

<170>  PatentIn version 3.5

<210>  1
<211>  9
<212>  PRT
<213>  Artificial

<220>
<223>  mERK2 136-144

<400>  1

Gln Tyr Ile His Ser Ala Asn Val Leu
1               5


<210>  2
<211>  9
<212>  PRT
<213>  Artificial

<220>
<223>  TRP-2

<400>  2

Ser Val Tyr Asp Phe Phe Val Trp Leu
1               5


<210>  3
<211>  9
<212>  PRT
<213>  Artificial

<220>
<223>  gp100

<400>  3

Glu Gly Ser Arg Asn Gln Asp Trp Leu
1               5


<210>  4
<211>  23
<212>  RNA

<213>  Artificial

<220>
<223>  m-miR298-5p

<400>  4
ggcagaggag ggcuguucuu ccc                                              23


<210>  5
<211>  22
<212>  RNA
<213>  Artificial

<220>
<223>  m-miR-298-3p

<400>  5
gaggaacuag ccuucucuca gc                                               22


<210>  6
<211>  23
<212>  RNA
<213>  Artificial

<220>
<223>  m-miR-1943-5p

<400>  6
aagggaggau cugggcaccu gga                                              23


<210>  7
<211>  21
<212>  RNA
<213>  Artificial

<220>
<223>  m-miR-1943-3p

<400>  7
caggugccag cuccucccuu c                                                21


<210>  8
<211>  22
<212>  RNA
<213>  Artificial

<220>
<223>  m-miR-5099-5p

<400>  8
guuagaaauu acauugauuu aa                                               22


<210>  9
<211>  20
<212>  RNA
<213>  Artificial

<220>

<223>  m-miR-5099-3p

<400>  9
uuagaucgau guggugcucc                                                    20


<210>  10
<211>  22
<212>  RNA
<213>  Artificial

<220>
<223>  m-miR-150-5p

<400>  10
ucucccaacc cuuguaccag ug                                                 22


<210>  11
<211>  22
<212>  RNA
<213>  Artificial

<220>
<223>  m-miR-150-3p

<400>  11
cugguacagg ccugggggau ag                                                 22


<210>  12
<211>  23
<212>  RNA
<213>  Artificial

<220>
<223>  m-miR-223-5p

<400>  12
cguguauuug acaagcugag uug                                                23


<210>  13
<211>  22
<212>  RNA
<213>  Artificial

<220>
<223>  m-miR-223-3p

<400>  13
ugucaguuug ucaaauaccc ca                                                 22


<210>  14
<211>  21
<212>  RNA
<213>  Artificial

<220>
<223>  m-miR-3470b-5p

<400>  14

ucacucugua gaccaggcug g                                                21

<210>  15
<211>  18
<212>  RNA
<213>  Artificial

<220>
<223>  m-miR-3470b-3p

<400>  15
ccugccucug ccucccga                                                    18